# EUROPEAN PATENT SPECIFICATION

(11) **EP 0 957 903 B1**
(45) Date of publication and mention of the grant of the patent: **10.08.2005**
(21) Application number: 97944319.9
(22) Date of filing: 18.09.1997
(51) Int. Cl.: A61K 31/045, A61P 17/00, A61P 25/28

(54) **DIOLS OF NORBORNENE AND NORBORNANE COMPOUNDS FOR TREATING PIGMENTATION DISORDERS, NEURODEGENERATIVE DISEASES OR PROLIFERATIVE SKIN DISORDERS**
NORBORNEN- UND NORBORNANDIOLEN ZUR BEHANDLUNG VON PIGMENTIERUNGSSTÖRUNGEN, NEURODEGENERATIVEN ERKRANKUNGEN ODER PROLIFERATIVEN HAUTERKRANKUNGEN
DIOLS DE NORBORNENE ET NORBORNANE POUR LE TRAITEMENT DES TROUBLES DE LA PIGMENTATION, DES MALADIES NEURODEGENERATIVES OU DES MALADIES PROLIFERATIVES DE LA PEAU

(30) Priority: 18.09.1996 US 26577 P; 21.01.1997 US 35947 P; 04.02.1997 US 36863 P; 04.06.1997 US 48597 P
(43) Date of publication of application: 24.11.1999
(73) Proprietor: Applied Genetics Incorporated Dermatics, Freeport, NY 11520 (US)
(72) Inventor: BROWN, David, A., Ellicott City, MD 21042 (US); KHORLIN, Alexander, A., North Potomac, MD 20878 (US); LESIAK, Krystyna, Gaithersburg, MD 20879 (US); REN, Wu, Yun, Germantown, MD 20874 (US)
(74) Representative: Froud, Clive
(86) International application number: PCT/US1997/016642
(87) International publication number: WO 1998/011882

(56) References cited:
- EP-A- 0 643 113
- WO-A-87/04617
- WO-A-89/00853
- WO-A-96/09810
- WO-A-97/03170
- WO-A-97/30692
- WO-A-98/55085
- WO-A-99/08654
- US-A- 4 102 995
- US-A- 4 390 532
- US-A- 4 528 126
- US-A- 5 041 485
- US-A- 5 190 978
- US-A- 5 352 440
- US-A- 5 434 144
- US-A- 5 525 635
- US-A- 5 532 001
- US-A- 5 626 839
- HASENFRATZ: "Experience in the treatment of acne vulgaris with the preparation acne-medice" ASTHETISCHE MEDIZIN, vol. 15, no. 6, 1966, pages 206-208, XP001070801

## Description

### BACKGROUND OF THE INVENTION

### 1. Field of the Invention

The present invention relates to the use of diols of norbornene or norbornane compounds in the preparation of a medicament for the treatment, prevention, or alteration of a disease or condition mediated by deficiencies in the NO/cGMP/PKG signal transduction pathway, which is selected from hypopigmentation disorders; skin proliferative disorders or disorders of keratinization; neurodegenerative disorders; heart disease, including stable angina pectoris, unstable angina, myocardial infarction, myocardial failure associated with myocardial ischemia, atherosclerosis, vascular hypertrophy and thrombosis; hypertension; stroke; primary pulmonary hypertension, chronic obstructive pulmonary disease and adult respiratory distress syndrome; microvascular functional abnormalities in diabetes; hemostatic irregularities of glomerular vascular and tubular function; disorders of bladder function and reflex relaxation for micturition; disorders of neurotransmitter release, neuron morphogenesis, synaptic plasticity and neuroendocrine regulation; regional pain, including migraine headaches; benign anal disease; impotence; inhibition of tumor growth; and stimulation of wound healing.

### 2. Description of Related Art

U.S. Patent 5,352,440 is directed to increasing melanin synthesis in melanocytes and increasing pigmentation by administration of certain diacylglycerol compounds.

U.S. Patent 5,532,001 is directed to increasing pigmentation in mammalian skin via administration of certain DNA fragments.

U.S. Patent 5,554,359 is directed to increasing levels of melanin in melanocytes by administration of lysosomotropic agents.

Krieger (Arzneimittel-Forschung, vol. 18, no. 3, 1968, pages 324 - 330) describes the medical use of 5-norbornene-2,2-dimethanol (structure XI-C) and 2,6-norbornanediol as choleretics.

### SUMMARY OF THE INVENTION

The present invention provides the use of one or more diols of norbornane or norbornene compounds as defined below in the preparation of a medicament for the treatment, prevention, or alteration of a disease or condition mediated by deficiencies in the NO/cGMP/PKG signal transduction pathway, which is selected from hypopigmentation disorders; skin proliferative disorders or disorders of keratinization; neurodegenerative disorders; heart disease, including stable angina pectoris, unstable angina, myocardial infarction, myocardial failure associated with myocardial ischemia, atherosclerosis, vascular hypertrophy and thrombosis; hypertension; stroke; primary pulmonary hypertension, chronic obstructive pulmonary disease and adult respiratory distress syndrome; microvascular functional abnormalities in diabetes; hemostatic irregularities of glomerular vascular and tubular function; disorders of bladder function and reflex relaxation for micturition; disorders of neurotransmitter release, neuron morphogenesis, synaptic plasticity and neuroendocrine regulation; regional pain, including migraine headaches; benign anal disease; impotence; inhibition of tumor growth; and stimulation of wound healing: 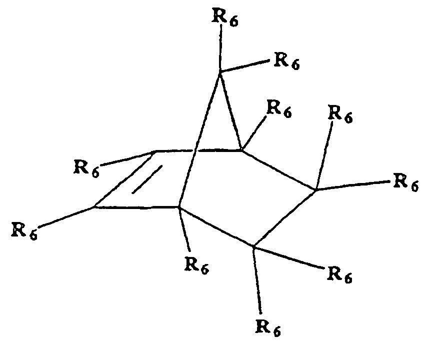 or 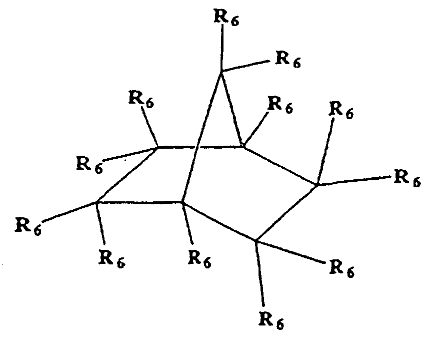 wherein
[each R₆ is independently selected from hydrogen; halogen; or a group containing from one atom to twenty atoms, one of which is carbon, nitrogen, oxygen, or sulfur; hydroxyl, hydroxymethyl, -(CH₂)ₙOH, -(CH₂)ₙOR₁, -(CH₂)ₙ-CH(OH)-CHOH, -(CH₂)ₙ-CH(OH)-CH(OH)R₁, -(CH₂)ₙ-CH(OH)-(CH₂)ₙ-CH₂(OH), -(CH₂)ₙ-CH(OH)-(CH₂)ₙ-CH(OH)R₁ or -CH₂OR₃, wherein each n is independently O or an integer of from 1-25;
each R₁ is independently selected from hydrogen; halogen; or a group containing from one atom to twenty atoms, one of which is carbon, nitrogen, oxygen, or sulfur;
each of R₃ is independently selected from hydrogen or an acyl or amino acyl group containing from one atom to twenty atoms, at least one of which is carbon, nitrogen, oxygen, or sulfur;
and pharmaceutically acceptable salts thereof.

### BRIEF DESCRIPTION OF THE DRAWINGS

Figures 1A-1D are printouts from an Oncor Imaging System™ of Fontana-Masson stained guinea pig skin biopsy samples as described in Example 5.
Figure 2 is a series of bar graphs depicting the structure activity results obtained in Example 7.
Figures 3A-3D are printouts of normal human epidermal melanocytes and melanosomes as described in Example 8.
Figures 4A-4B are printouts as described in Example 10.

### DETAILED DESCRIPTION OF THE INVENTION

The present invention is based on the unique observation that certain compounds effectively and efficiently induce melanogenesis in mammalian cells, which has several consequences. First, increasing melanogenesis leads to increasing the melanin content of melanocytes, and hence results in increased pigmentation or darkened color of the skin, hair wool or fur. Thus, the present invention is useful in the treatment of hypopigmentation disorders, such as albinism, vitiligo, etc. It is also believed that increasing the pigmentation of skin according to the present invention will protect such skin from subsequent UV light damage, sunburn, photoaging and development of skin cancers. Finally, since the compositions described herein induce differentiation of a melanoma cell line, the present invention may be used to treat hyperproliferative disorders such as actinic keratosis, basal cell carcinoma, squamous cell carcinoma, fibrous histiocytoma, dermatofibrosarcoma protuberans, hemangioma, nevus flammeus, xanothoma, Kaposi's sarcoma, mastocytosis, mycosis fungoides, lentigo, nevocellular nevus, lentigo maligna, malignant melanoma, and metastatic carcinoma.

The present compositions are also useful in the treatment of diseases characterized by inflammation and disturbance of keratinization, including psoriasis vulgaris, psoriasis eosinophilia, acne vulgaris, acne conglobata, acne fulminans, osteoma cutis, nodulocystic acne, and cystic acne.

The compounds also effectively and efficiently increase differentiation of neuronal cells, including increased neuronal dendricity and neuronal tyrosine hydroxylase activity, which has several consequences. First, increasing dendricity leads to increased neuronal communication, thereby increasing neuronal function and performance. Thus, the present invention is useful for treating diseases or disorders marked by reduction of neuronal dendricity and function, including but not limited to Parkinson's disease, amyotrophic lateral sclerosis, Alzheimer's disease, or any other neurodegenerative disease, or physical or toxic damage to brain, spinal or peripheral nerve cells. Further, the present invention is useful for restoring or optimizing neuronal communication, function or performance.

Second, increasing tyrosine hydroxylase activity directly increases dopamine synthesis. Thus, the present invention is particularly useful for treating Parkinson's disease which is specifically marked by depletion of dopamine synthesis.

Third, induction of neuronal differentiation reverses neuronal proliferative disorders. Thus, the present invention is useful for treating neuronal proliferative, tumorous, or cancerous disorders, or said disorders in any other cell type that might be similarly affected.

Finally, since the compositions described herein induce differentiation, dendricity and tyrosine hydroxylase in a neuronal cell model, the present invention is useful for treating additional neurodegenerative disorders or neuropathies including but not limited to diffuse cerebral cortical atrophy, Lewy-body dementia, Pick disease, mesolimbocortical dementia, thalamic degeneration, Huntington chorea, cortical-striatal-spinal degeneration, cortical-basal ganglionic degeneration, cerebrocerebellar degeneration, familial dementia with spastic paraparesis, polyglucosan body disease, Shy-Drager syndrome, olivopontocerebellar atrophy, progressive supranuclear palsy, dystonia musculorum deformans, Hallervorden-Spatz disease, Meige syndrome, familial tremors, Gilles de la Tourette syndrome, acanthocytic chorea, Friedreich ataxia, Holmes familial cortical cerebellar atrophy,
Gerstmann-Straussler-Scheinker disease, progressive spinal muscular atrophy, progressive balbar palsy, primary lateral sclerosis, hereditary muscular atrophy, spastic paraplegia, peroneal muscular atrophy, hypertrophic interstitial polyneuropathy, heredopathia atactica polyneuritiformis, optic neuropathy, and ophthalmoplegia.

It has also been discovered that the present class of compounds have their action blocked by scavengers of nitric oxide (NO), and by inhibitors of cyclic guanosine monophosphate (cGMP) or inhibitors of cGMP-activated protein kinase (PKG). This indicates that these compounds act via the NO/cGMP/PKG signal transduction pathway. Unlike previous compounds like nitroglycerin and isosorbide dinitrate that stimulate this pathway by releasing NO upon reaction with intracellular sulfhydryl groups (Smith and Reynard, 1992, Pharmacology, W. B. Saunders Co., Philadelphia, PA, pp. 626-31), the compounds used in this invention appear to act by direct stimulation of nitric oxide synthase (NOS) activity, thus generating NO *de novo*. Whereas depletion of intracellular sulfhydryl groups rapidly leads to tolerance and ineffectiveness of nitroglycerin and related compounds (Smith and Reynard 1992), tolerance will not be acquired to the compound used in the present invention since they do not require the presence of sulfhydryl groups for generation. Thus, it is contemplated that the compounds used in the present invention will provide a preferred alternative method of treatment for conditions presently treated by NO donors.

Both clinical application and research studies have demonstrated that stimulation of the NO/cGMP/PKG pathway is useful for treatment of:
(i) heart disease including stable angina pectoris, unstable angina, myocardial infarction, and myocardial failure associated with myocardial ischemia, atherosclerosis, vascular hypertrophy, and thrombosis (Cooe and Dzau, 1997, *Annu. Rev. Med*. 48:489-509; Thadani, 1997, *Cardiovasc. Drugs* 10:735);
(ii) hypertension (Cooe and Dzau, 1997);
(iii) stroke (Samdani, *et al*., 1997, Stroke 28:1283-1288);
(iv) primary pulmonary hypertension, chronic obstructive pulmonary disease, and adult respiratory distress syndrome (Adnot and Raffestin, 1996, *Thorax* 51:762-764; Marriott and Higenbottam, 1997, *Schweiz Med. Wochenschr*. 127:709-714);
(v) microvascular functional abnormalities in diabetes that link insulin-resistance to hypertension, thrombosis and atherosclerosis (Tooke, *et al*., 1996, *Diabetes Res. Clin. Pract*. 31Suppl:S127-S132; Baron, 1996, *J. Investig. Med*. 44:406-412);
(vi) hemostatic irregularities of glomerular vascular and tubular function with consequences for development of hypertension (Kone and Baylis, 1997, *Am. J. Physiol*. 10:F561-578; *Am. J. Hypertens*. 10:129-140);
(vii) disorders of bladder function and reflex relaxation for micturition (Andersson, 1996, *Curr. Opin. Obstet. Gynecol*. 8:361-365);
(viii) disorders of neurotransmitter release, neuron morphogenesis, synaptic plasticity, and neuroendrocrine regulation (Dawson and Dawson, 1996, Neurochem. *Int.* 29:97-110; Brann, *et al*., 1997, *Neuroendocrinology* 65:385-395);
(ix) regional pain including migraine headaches (Mashimo, *et al*., 1997, *J. Clin. Pharmacol*. 37:330-335; Packard and Ham, 1997, Mar. 37:142-152);
(x) gastrointestinal protection from non-steroidal anti-inflammatory drugs (Rishi, *et al*., 1996, *Indian J. Physiol. Pharmacol*. 40:377-379) ;
(xi) benign anal disease (Gorfine, 1995, *Dis. Colon Rectum* 38:453-456);
(xii) impotence (Andersson and Stief, 1997, *World J. Urol*. 15:14-20);
(xiii) regulation of tissue free radical injury (Rubbo, *et al*., 1996, *Chem. Res. Toxicol.* 9:809-820); and
(xiv) inhibition of tumor growth, tumor apoptosis, angiogenesis, and metastasis (Pipili-Synetos, *et al*., 1995, *Br. J. Pharmacol*. 116:1829-1834; Xie, *et al*., 1996, *J. Leukoc. Biol*. 59:797-803); and
(xv) stimulation of wound healing including cuts, tendon injury and thermal injury (Schaffer, *et al*., 1996, *J. Surg. Res.* 63:237-240; Murrell, *et al*., 1997, *Inflamm. Res*. 46:19-27; Carter, *et al*., 1994, *Biochem. J.* 304 (Pt 1):201-04).

In addition, the NO/cGMP/PKG pathway mediates melanogenesis induced by ultraviolet light (Romero-Graillet, *et al*., 1996, *J. Biol. Chem*. 271:28052-28056; Romero-Graillet, *et al*., 1997, *J. Clin. Invest*. 99:635-642) and aliphatic and alicyclic diols (United States patent application S.N., entitled "Dermatalogical Compositions and Methods" filed concurrently herewith).

The active compounds used in the present invention have the structures described above. More preferably each of **R**_{**1**} is independently selected from hydrogen; fluoro; chloro; or C₁-C₂₀ alkyl, C₂-C₂₀ alkenyl, C₂-C₂₀ alkynyl, C₇-C₂₀ aralkyl, C₈-C₂₀ aralkenyl, C₈-C₂₀ aralkinyl, or C₆-C₂₀ aryl, each of which may contain one or more different heteroatoms or heteroatoms of the same type, or carboxyl, carboxamido, carbalkoxy, sulfamido, sulfonamido; hydroxyl, or amino. More preferably each of **R**_{**3**} is independently selected from hydrogen or C₁-C₁₈ acyl, which may contain one or more different heteroatoms or heteroatoms of the same type.

The preparation of the present compounds would be apparent to one of ordinary skill, and many of them are commercially available. Representative preferred compounds include, but are not limited to:
5-norbornene-2,2-dimethanol
norbornane-2,2-dimethanol
2,3-norbornanediol (exo or endo or cis or trans)
2,3-cis-exo-norbornanediol
2-endo-hexadecylamino-5-norbornene-2,3-exo-dimethanol
2-(propyl-1,2-diol)-norbornane

Particularly preferred compounds used in this invention are 5-norbornene-2,2-dimethanol; norbornane-2,2-dimethanol; 2,3-cis-exo-norbornanediol, 2-(propyl-1,2-diol)-norbornane.

The compositions used in the present invention contemplate the use of one or more of the above-mentioned compounds as an active ingredient for various uses. In a preferred embodiment, the active ingredient(s) is combined with an acceptable carrier to form a topical formulation which may be placed on the skin for dermatological uses. Topical formulations may include ointments, lotions, pastes, creams, gels, drops, suppositories, sprays, liquids, shampoos, powders and transdermal patches. Thickeners, diluents, emulsifiers, dispersing aids or binders may be used as needed. Preferably, one function of the carrier is to enhance skin penetration of the active ingredient(s), and should be capable of delivering the active ingredient(s) to melanocytes under in vivo conditions. Suitable carriers are well known to one of ordinary skill, and include liposomes, ethanol, dimethylsulfoxide (DMSO), petroleum jelly (petrolatum), mineral oil (liquid petrolatum), water, dimethylformamide, dekaoxyethylene-oleylether, oleic acid, 2-pyrrolidone and Azone® brand penetration enhancer (Upjohn). A particularly preferred composition includes an active ingredient(s) as described above, with one of 2-pyrrolidone, oleic acid and/or Azone® as penetration enhancer, solubilized in a base of water, ethanol, propanol and/or propylene glycol (the latter component having properties of a carrier, penetration enhancer and an active ingredient as described herein). Depending on the specific application, the compositions of the present invention may also include other active ingredients, as well as inert or inactive ingredients.

The dose regimen will depend on a number of factors which may readily be determined, such as severity and responsiveness of the condition to be treated, but will normally be one or more doses per day, with a course of treatment lasting from several days to several months, or until a cure is effected or a diminution of disease state is achieved, or a cosmetically desired degree of melanogenesis (tanning) is achieved, depending on the application. One of ordinary skill may readily determine optimum dosages, dosing methodologies and repetition rates. In general, it is contemplated that topical formulations (such as creams, lotions, solutions, etc.) will have a concentration of active ingredient of from about 0.01% to about 50%, preferably from about 0.1% to about 10%. In general, it is contemplated that unit dosage form compositions according to the present invention will contain from about 0.01 mg to about 100 mg of active ingredient, preferably about 0.1 mg to about 10 mg of active ingredient.

Another aspect of the present invention is based on the observation that the subject compounds which stimulate melanin production act via the Nitric Oxide/cyclic Guanosine monophosphate/Protein Kinase G ("NO/cGMP/PKG") pathway. Thus, the present invention includes the compounds described above, which act via the NO/cGMP/PKG pathway to stimulate melanin synthesis by increasing cellular production of NO, cGMP or PKG. Conversely, agents which decrease cellular production of NO, cGMP or PKG will decrease or suppress melanin production and pigmentation in mammalian skin, hair, fur or wool. Such is useful in, for example, the lightening of skin, hair, wool or fur for cosmetic purposes, or the treatment of hyperpigmentation or uneven pigmentation disorders such as vitiligo, dermal melanocytosis, Franceschetti-Jadassohn Syndrome, etc. For such depigmentation applications, the formulation and dosing would be as described above with respect to pigmentation applications.

Discovery of the pathway through which the present compounds act also leads to methods for screening compounds for melanogenic activity and potency, or for their ability to reduce or suppress melanogenesis, based on measurement of generation of nitric oxide (NO) or measurement of nitric oxide synthesis (NOS) activity. Methods for measurement of NO or NOS include but are not limited to the following well known methods. Measurement of NO is usually based on the fact that NO rapidly decomposes to nitrate and nitrite in aqueous solution. Nitrate reductase is added to culture media or cell extracts to ensure complete conversion of nitrate to nitrite. Griess reagents (sulfanilamide and N-[1-naphthyl]-ethylenediamine) are then added to convert nitrite into a deep purple azo compound that absorbs maximally at 540 nm (Schmidt, *et al*., 1995, *Biochemica* 2:22). Reactions are typically carried out in a 96-well format with absorbances read on a microtiter plate reader. Alternatively, following conversion of nitrate to nitrite as described above, DAN reagent (2,3-diaminonaphthalene) is added followed by NaOH which converts nitrite into the fluorescent compound 1(H)-naphthotriazole. This is measured fluorimetrically with excitation at 365 nm and emission at 450 nm, typically in a 96-well format (Miles, *et al*., 1995, *Methods* 7:40). NOS activity is measured by adding [³H]-arginine to intact tissues or protein extracts, and measuring release of ³H resulting from the conversion of arginine to citrulline during the enzymatic formation of NO by NOS (Baudouin and Tachon, 1996, *J. Invest. Dermatol*. 106:428-431). Alternatively, the production of cGMP or activity of PKG can be used as a screening tool. cGMP may be measured by commercially available immunoassay (see Romero-Graillet, *et al*., 1996, *J. Biol. Chem*. 271:28052-28056). PKG may be measured by cyclic GMP dependent kination of a primary histone target (see Hidaka, *et al., Biochemistry* 1984, 23, 5036-5041)

The use of and useful and novel features of the present compositions will be further understood in view of the following non-limiting examples.

### Example 1 (does not form part of the invention)

The Cloudman S91 mouse melanoma cell line was obtained from American Type Culture Collection (ATCC). Cells were cultured in Dulbecco's Modified Eagles Medium (DMEM) containing 10% calf serum, 2 mM L-glutamine, 10 U Penicillin/ml and 10 ug Streptomycin/ml according to a previously published protocol (Eller, *et al*., Proc. Natl. Acad. Sci. 93:1087-92. 1996). For testing propylene glycol and analogues for induction of melanogenesis, S91 cells were plated at 10⁵ cells/35 mm dish in 10% calf serum. One day after plating, media was removed and replaced with media containing 2% calf serum and test compounds (Eller, *et al*., 1996). Cells were cultured for 6 days at 37°C in 5% CO₂ in a humidified incubator. Following this treatment period, cells were examined microscopically and the portion of dedifferentiated and differentiated cells was estimated. Previous studies have shown that dedifferentiated S91 cells have a rounded, spindly appearance while differentiated S91 cells have a flattened, cuboidal, multipolar and dendritic appearance (Orlow, *et al*., *Exp. Cell Res.* 191:209-218, 1990).

Following this microscopic examination, cells were detached from dishes by trypsin. The time required for detachment by trypsin was recorded as an additional indicator of the phenotypic effects of test compounds. For each treatment, a subsample of cells was counted to determine the effects of treatment compounds on cellular proliferation. The remainder of cells were used for determination of melanin content. Melanin was extracted from cells by vortexing for 15 min in 1N NaOH. Standards were prepared by dissolving melanin (Sigma) in 1 N NaOH (Eller, *et al*., 1996). Absorbance of standards and samples was measured at 475 nm. Melanin was expressed as pg melanin/cell.

Tables 1 and 2 below show the results obtained when testing formulations containing various concentrations of 1,2-propanediol as the active ingredient. In the control, no test compound was added to the medium.

**TABLE 1**

| Concentration | Cells (x10⁶) | ug Melanin | pg Melanin/Cell |
|---|---|---|---|
| Control | 0.48 | 2.52 | 5.3 |
| 1% (136 mM) | 0.52 | 4.88 | 9.4 |
| 2% (272 mM) | 0.50 | 6.24 | 12.5 |
| 3% (408 mM) | 0.20 | 4.03 | 20.2 |
| 4% (544 mM) | 0.10 | 4.01 | 40.1 |
| 5% (680 mM) | 0.08 | 2.31 | 28.9 |

**TABLE 2**

| Morphology | | | |
|---|---|---|---|
| Concentration | Rounded Spindly | Flattened Cuboidal | Trypsinization Detachment Time |
| Control | 100% | | ≤3 min |
| 1% (136 mM) | 90% | 10% | ≤6 min |
| 2% (272 mM) | 70% | 30% | ≤9 min |
| 3% (408 mM) | 40% | 60% | ≤12 min |
| 4% (544 mM) | 15% | 85% | ≤15 min |
| 5% (680 mM) | | 100% | ≤15 min |

### EXAMPLE 2 (does not form part of the invention)

The same procedure as in Example 1 was followed, except that ethanol, and isomers of propanediol and butanediol were used as test compounds. The results are set forth in Tables 3 and 4. The data demonstrate that several isomers of propanediol and butanediol induce melanogenesis and differentiation of S91 melanoma cells. Both 50 mM propanediol (PG) or butanediol (BD) resulted in an approximate 1.5-fold increase of melanogenesis, while 150 mM resulted in about a 2-fold increase following a single treatment. Whereas 1,2 propanediol (PG-1,2) and (S)-(+)-1,2-Propanediol (PG-S-1,2) resulted in no reduction of cell proliferation at the levels used in this experiment, 150 mM 1,3-propanediol (PG-1,3), 2,3-butanediol (BD-2,3) or 1,3-butanediol (BD-1,3) resulted in a reduction of cell numbers by one-third. In addition, the butanediols appeared to result in greater differentiation of S91 cells than the propanediols, as evidenced by earlier and greater morphological changes, and in the case of BD-2,3, a more adherent phenotype. Ethanol (EtOH) had no effect on cells at 340 mM but was toxic at 850 mM as indicated by low cell survival. Ethanol did not induce melanogenesis at any concentration tested. Glycerol (G) had only a slight effect on melanogenesis and differentiation at the concentrations tested in this experiment, indicating that triols may be less effective inducers of these phenotypes than diols.

**TABLE 3**

| | Cells(x10⁶) | ug Melanin | pg Melanin/Cell |
|---|---|---|---|
| Control | 0.100 | 1.17 | 11.7 |
| 1.0% ETOH¹ | 0.104 | 1.14 | 11.0 |
| 2.0% ETOH² | 0.100 | 1.25 | 12.5 |
| 5.0% ETOH³ | 0.032 | 0.17 | 5.3 |
| 50 mM PG-1,2 | 0.084 | 1.31 | 15.6 |
| 150 mM PG-1,2 | 0.072 | 1.73 | 24.0 |
| | | | |
| 50 mM PG-S-1,2 | 0.088 | 1.51 | 17.1 |
| 150 mM PG-S-1,2 | 0.080 | 2.04 | 25.5 |
| 50 mM PG-1,3 | 0.064 | 1.31 | 20.4 |
| 150 mM PG-1,3 | 0.044 | 1.04 | 23.6 |
| 50 mM G | 0.092 | 1.03' | 11.2 |
| 150 mM G | 0.084 | 1.09 | 13.0 |
| | | | |
| 50 mM BD-2,3 | 0.072 | 1.12 | 15.6 |
| 150 mM BD-2,3 | 0.040 | 0.95 | 23.8 |
| 50 mM BD-1,3 | 0.064 | 0.99 | 15.5 |
| 150 mM BD-1,3 | 0.048 | 0.87 | 18.1 |

| | | | |
|---|---|---|---|
| ¹170 mM | | | |
| ²340 mM | | | |
| ³850 mM | | | |

**TABLE 4**

| Morphology | | | |
|---|---|---|---|
| | Rounded Spindly | Flattened Cuboidal | Trypsinization Detachment Time |
| Control | 100% | | 3 min |
| 1.0% ETOH | 100% | | 3 min |
| 2.0% ETOH | 100% | | 3 min |
| 5.0% ETOH | 100% | | 3 min |
| 50 mM PG-1,2 | 75% | 25% | 3 min |
| 150 mM PG-1,2 | 50% | 50% | 6 min |
| | | | |
| 50 mM PG-S-1,2 | 75% | 25% | 3 min |
| 150 mM PG-S-1,2 | 50% | 50% | 6 min |
| 50 mM PG-1,3 | 75% | 25% | 3 min |
| 150 mM PG-1,3 | 50% | 50% | 6 min |
| 50 mM G | 100% | | 3 min |
| | | | |
| 150 mM G | 75% | 25% | 3 min |
| 50 mM BD-2,3 | 25% | 75% | 3 min |
| 150 mM BD-2,3 | | 100% | 9 min |
| 50 mM BD-1,3 | 25% | 75% | 3 min |
| 150 mM BD-1,3 | | 100% | 6 min |

Melanogenesis is the most characteristic feature of melanocyte differentiation (*J. Cell Sci.* 107:1095-1103, 1994), and, is inversely correlated with rate of proliferation in melanoma cell lines (*Neoplasia* 31:545-9, 1984; *Biochem. Biophys. Res. Commun*. 177:545-50, 1991; *Exp. Dermatol*. 4:192-198, 1995). As a general rule, increased proliferation commensurate with dedifferentiation are hallmarks of rapid tumor progression and a poor prognosis, while decreased proliferation and differentiation are indicative of more long-term survival (*Introduction to the Cellular and Molecular Biology of Cancer,* L. M. Franks and N. Teich, 1987, Oxford University Press). Thus, the ability of the present compounds to induce melanogenesis and slow cell growth is indicative of their ability to act as chemotherapeutic agents. Induction of melanogenesis combined with a reduced rate of cellular proliferation is indicative of induction of differentiation in S91 cells. In addition, the change of cellular morphology from a rounded, spindly appearance to a flattened, cuboidal appearance is further indication of differentiation in S91 cells (*Exp. Cell Res.* 191:209-218, 1990). Thus, the compounds of the present invention are not only tanning agents, but also chemotherapeutic agents capable of delaying tumor progression and increasing long-term survival.

It should be noted that the effects of propylene glycol (Example 1) and related diols and triols (Examples 1 & 2) on S91 cells are identical to those resulting from treatment of S91 cells with retinoids; that is, induction of melanogenesis, induction of differentiation, increased adherence, and inhibition of proliferation (Laukharanta, *et al., Arch. Dermatol. Res.* 277:147-150, 1985). Given this similarity of biological responses, it is believed that the agents described herein are effective in treating those disorders presently treated with the retinoids including a variety of forms such as psoriasis, acne and dermatoses.

### EXAMPLE 3

The same procedures as in Examples 1 and 2 were followed to examine the effect of additional compounds on melanogenesis in S91 cells. The results described in Table 5 show the concentration of a number of compounds required to induce 2-fold or greater melanization in S91 cells. Many compounds are more potent than those described in Examples 1 and 2. For example, 2,3-pyridinediol was potent at 100 uM; 1,4-dioxane-2,3-diol and β-estradiol at 500 uM; 5-norbornene-2,2-dimethanol at 5 mM; 3,3-dimethyl-1,2-butanediol and 1,2-cis-cyclopentanediol at 10 mM; and 2,3-dimethyl-2,3-butanediol at 25 mM. All of the compounds listed in Table 5 except 1,4-dioxane-2,3-diol, induced transformation of S91 cells from a rounded bipolar morphology to a flattened cuboidal multipolar morphology concomitant with induction of melanogenesis; this indicates their potential usefulness as chemotherapeutic agents that act by inducing differentiation of tumor cells. All of the compounds listed in Table 5 except 5-norbornene-2,2-dimethanol, β-estradiol, and 2,3-pyridinediol induced increased trypsinization time concomitant with induction of melanogenesis; alterations of adherence properties are related to changes of metastatic potential of tumor cells.

**TABLE 5**

| Concentration Required for ≥2-fold | |
|---|---|
| Compound | Melanin Induction in S91 Cells |
| 2,3-Pyridinediol* | 100 uM |
| 1,4-Dioxane-2,3-Diol* | 500 uM |
| β-Estradiol* | 500 uM |
| 5-Norbornene-2,2-Dimethanol | 5 mM |
| 1,2-cis-Cyclopentanediol* | 10 mM |
| 3,3-Dimethyl-1,2-Butanediol* | 10 mM |
| 2,3-Dimethyl-2,3-Butanediol* | 25 mM |
| 1,2-*trans*-Cyclopentanediol* | 50 mM |
| 2-Methyl-1,3-Propanediol* | 50 mM |
| 2,3-Butanediol* | 100 mM |
| 1,2-Propanediol* | 150 mM |

| | |
|---|---|
| * comparative compounds | |

Comparative compounds in addition to those described in Examples 1 and 2, that did not induce significant (≥2-fold increase) melanogenesis in S91 cells when tested over a range of concentrations up to a toxic dose included: 1-propanol; 2-propanol; oleic acid; 2-phenyl-1,2-propanediol; 1,3-cyclohexanediol; tartaric acid; ascorbic acid; Azone®, 2-pyrrolidone; D-ribose; 2-deoxy-D-ribose; N-methyl-D-glucamine; hydroxymethyl uracil; and tetrabutylammonium chloride. Of these compounds, only 2-pyrrolidone resulted in profound morphological differentiation of S91 cells, indicating that it may augment melanogenesis and/or exert antitumorigenic activity in the absence of melanogenesis.

The PKC inhibitors H7 (1-[5-isoquinolinyl-sulfonyl]-2-methyl-piperazine) and D-sphingosine also induced melanogenesis in S91 cells. In addition, these PKC inhibitors enhanced melanogenesis induced by propylene glycol in S91 cells. These results indicate that propylene glycol does not induce melanogenesis by induction of PKC, or require PKC for induction of melanogenesis.

### EXAMPLE 4

Normal human epidermal melanocytes (NHEMs) were examined for induction of melanogenesis using cells and media from Clonetics Corporation (San Diego, California). Cells were cultured exactly as specified by the supplier. Based on induction of a 1.5-fold increase of melanin in NHEMs, the most potent compound examined was 2,3-pyridine-diol at 200 uM, followed by 5-norbornene-2,2-dimethanol at ≤5 mM, 3,3-dimethyl-1,2-butanediol at 12.5 mM, and 2,3-dimethyl-2,3-butanediol and 1,2-*cis*-cyclopentanediol at 50 mM (Table 6). D-Ribose was inactive in NHEMs when tested over a range of concentrations up to a toxic dose. These results show that compounds of the present invention that exhibit activity in S91 cells, also exhibit activity in normal human melanocytes.

**TABLE 6**

| Concentration Required for ≥1.5-fold | |
|---|---|
| Compound | Melanin Induction in NHEMs |
| 2,3-Pyridinediol* | 200 uM |
| 5-Norbornene-2,2-Dimethanol | 5 mM |
| 3,3-Dimethyl-1,2-Butanediol* | 12.5 mM |
| 1,2-*cis*-cyclopentanediol* | 50 mM |
| 2,3-Dimethyl-2,3-Butanediol* | 50 mM |
| 1,2-Propanediol* | 150 mM |

| | |
|---|---|
| * comparative compounds | |

### EXAMPLE 5

Compounds were tested for melanogenic activity *in vivo* by application to American short-haired guinea pigs. Treatment sites were created by removal of fur using Nair® brand depilatory. Compounds were applied in 25 µl volumes twice per day for 5 days to each treatment spot as indicated in Table 7. In the Table, the numbers presented are the relative melanogenesis rating (mean ± SE), and are arranged according to the relative location on the animal, with the head being to the left and the tail being to the right. Propylene glycol (PG=13.6M), 2,3-butanediol (2,3-BD=10.95M), and 1,2-cis-cyclopentanediol (1,2-cs-CPD=10.7M) were applied as full strength solutions. 3,3-dimethyl-1,2-butanediol (3,3-M-1,2-BD) was applied as a 4M solution dissolved in ethanol. Two weeks following cessation of treatments, the degree of pigmentation was subjectively rated according to the following scale:

| | |
|---|---|
| 0 | no change |
| 0.5 | slight darkening, not easily discernible |
| 1 | slight darkening, easily discernible |
| 2 | moderate, even darkening |
| 3 | substantial, even darkening |
| 4 | profound, even darkening |

The results presented below showed that there was a progressive diminution of response to tanning agents from head to tails of animals. The magnitude of this diminished response was 3- to 4-fold. Thus, comparisons between treatment compounds were done relative to similar locations on the body of guinea pigs. Propylene glycol resulted in significant melanogenesis relative to depilitory treated controls located at the same relative body position. 2-methyl-1,3-propylene glycol and 2,3-butanediol were only slightly better melanogenic agents than propylene glycol. However, 3,3-dimethyl-1,2-butanediol and 1,2-cis-cyclopentanediol resulted in 4.5-fold and 5.5-fold greater melanogenesis than PG applied at similar body locations.

**TABLE 7**

| (does not form part of the invention) | | | |
|---|---|---|---|
| Treatment | | | |
| PG, 5 Days (n=6): | | | |
| 1.04 ± 0.21 | 0.83 ± 0.17 | 0.25 ± 0.09¹ | 0.33 ± 0.16¹ |
| | | | |
| 5 days (n=3): | | | |

| 2-M-PG | 2,3-BD | 2-M-PG | 2,3-BD |
|---|---|---|---|
| 1.25 ± 0.52 | 1.33 ± 0.17² | 0.58 ± 0.08² | 0.25 ± 0.14 |
| | | | |
| 5 Days (n=3): | | | |

| Nair | PG | 3,3-M-1,2-BD | 1,2-cs-CPD |
|---|---|---|---|
| 0² | 0.50 ± 0.25 | 1.16 ± 0.66² | 1.83 ± 0.33² |

| | | | |
|---|---|---|---|
| ¹P<0.05 relative to PG-treated site located nearest head in first row | | | |
| ²P<0.05 relative to PG-treated site in first row that is located at same position relative to head and tail | | | |

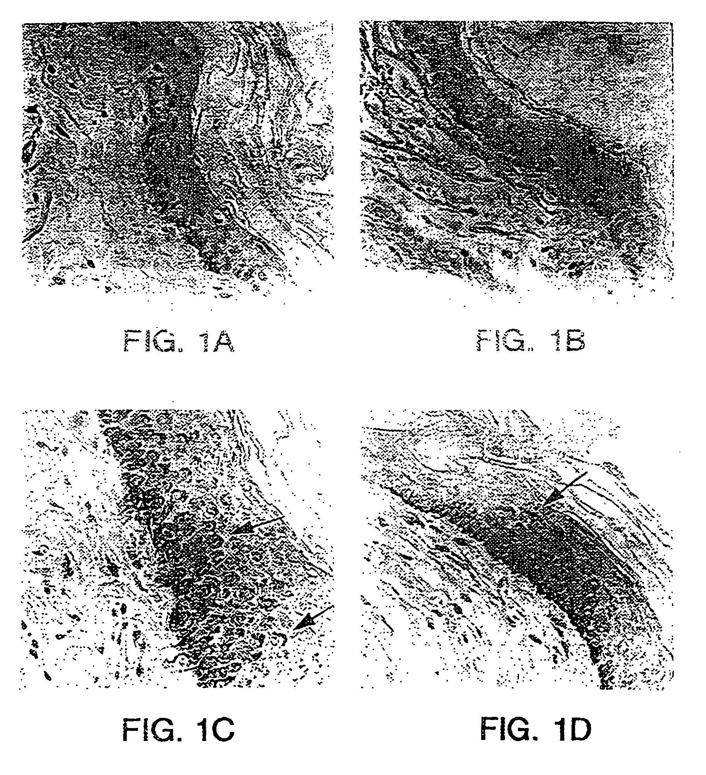

In order to minimize the effects of dimunition of response from head to tails of animals, all future experiments were done using only treatment spots located towards the tails of animals. Deemed as additionally beneficial, in this area of the animal differences of responsiveness to strong and weak inducers of pigmentation, as deduced from cell culture, were greatest. Comparison of the pigmentation ratings of these treatment spots showed the following descending order of induction: 8.7M 1,2-*cis*-cyclopentanediol (1,2-cs-CPD) > 4M 3,3-dimethyl-1,2-butanediol (3,3-M-1,2-BD) > a mixture of 8.5M 1,2-propylene glycol (1,2-PG)/1M 5-norbornene-2,2-dimethanol (5-NBene-2,2-DM)/2% 2-pyrrolidone (2-P; a penetration enhancer) > 1M 5-NBene-2,2-DM/2% 2P, > 11.3M 2-methyl-1,3-propylene glycol (2-M-1,2-PG) (Table 8; Figure 1A: untreated; 1B: 10.6M 1,2-PG/2% 2-P; 1C: 8.7M 1,2-cs-CPD; 1D: 1M 5NBene-2,2-DM/8.5M 1,2-PG/2% 2-P). In this region of the animals, responses to 13.61M 1,2-PG; 10.6M 1,2-PG/2% 2P, and 11M 2,3-dimethyl-2,3-butanediol were not significantly different from control (Nair or 2% 2P treated) spots. Pigmentation ratings were corrected for background (control treatment spots), normalized to 1M to account for the different amounts of each agent applied, and then normalized to results for 1,2-PG (Table 8). This comparison showed that the descending order of induction was 5-NBene-2,2-DM > 1,2-cs-CPD > 2-M-1,3-PG, and, that using 1,2-PG as carrier for 5-NBene-2,2-DM (Figure 1D) increased responsiveness to this compound. It is anticipated that further improvements in formulation will additionally improve responsiveness to 5-NBene-2,2-DM and other compounds in this invention. Biopsies results (Figure 1) showed that induction of melanogenesis was marked by deposition of melanin in keratinocytes, in some cases with formation of "supranuclear caps" (arrows, Figure 1C & 1D) indicative of induction of true natural UV-protective melanogenesis (Gates, R. R., and A. A. Zimmermann, 1953 *J. Invest. Dermatol*. 21:339-348), and a complete absence of inflammation, fibrosis or any other form of tissue damage.

**Table 8**

| Treatment | Pigmentation Rating | Background Corrected | Normalized to 1M | Normalized to 1,2-pG |
|---|---|---|---|---|
| *No Penetration Enhancer* | | | | |
| Nair | 0.08 ± 0.05 | 0 | | |
| | (n=6) | | | |
| | | | | |
| 13.61M | 0.29 ± 0.09 | 0.21 ± 0.03 | 0.015 ± 0.002 | 1.0 ± 0.1 |
| 1,2-PG** | (n=12) | | | |
| | | | | |
| 11.0M | 0.25 ± 0.14 | 0.17 ± 0.09 | 0.015 ± 0.008 | 1.0 ± 0.6 |
| 2,3-M-2,3-BD** | (n=3) | | | |
| | | | | |
| 11.3M | 0.58 ± 0.08* | 0.50 ± 0.07 | 0.044 ± 0.006 | 2.9 ± 0.4 |
| 2-M-1,3-PG** | (n=3) | | | |
| | | | | |
| 8.7M | 1.89 ± 0.27* | 1.75 ± 0.25 | 0.202 ± 0.029 | 13.5 ± 1.9 |
| 1,2-cs-CPD** | (n=9) | | | |
| | | | | |
| 4.0M | 1.17 ± 0.44* | 1.09 ± 0.41 | 0.272 ± 0.102 | 18.1 ± 6.8 |
| 3,3-M-1,2-BD** | (n=3) | | | |

| *Penetration Enhancer 2% 2-Pyrrolidone* | | | | |
|---|---|---|---|---|
| 2P | 0.17 ± 0.08 | 0 | | |
| | (n=6) | | | |
| | | | | |
| 10.6M | 0.33 ± 0.05 | 0.16 ± 0.02 | 0.015 ± 0.002 | 1.0 ± 0.15 |
| 1,2-PG/2P** | (n=6) | | | |
| | | | | |
| 1.0M | 0.66 ± 0.05* | 0.49 ± 0.04 | 0.490 ± 0.037 | 32.7 ± 2.5 |
| 5-NBene-2,2-DM/2P | (n=6) | | | |
| 8.5M | 1.00 ± 0.13* | 0.83 ± 0.11 | 0.670 ± 0.087¹ | 44.7 ± 5.8 |
| 1,2-PG/2P/1.0M 5-NBene-2,2-DM | (n=6) | | | |

| | | | | |
|---|---|---|---|---|
| *P<0.05; Students T-test | | | | |
| **comparative compounds | | | | |
| ¹Further background corrected for pigmentation induced by 1,2-PG/2P (0.16) | | | | |

### Example 6

Compounds were examined for their ability to induce tyrosinase activity in S91 mouse melanoma cells. Tyrosinase is the rate limiting enzyme in the melanogenic pathway. Its measurement provides a highly specific and sensitive indication of degree of induction of melanogenesis by test compounds. All cell culture conditions and treatments were as described above in Examples 1-3. Following treatments, cells were trypsinized, counted by Coulter, pelleted by centrifugation at 1000 X g, and analyzed for tyrosinase activity using modifications of previously described procedures (Pomerantz, S. H., 1966, *J. Biol. Chem.* 241:161-168; Jara, *et al*., 1988, *Pigment Cell Res.* 1:332-339.). Briefly, cell pellets were solubilized by sonicating for 5 seconds in 600 ul 50 mM phosphate buffer pH 6.8 containing 0.5% Triton-X100, followed by vortexing, incubation on ice for 30 min, and then revortexing. From this, 200 ul aliquots were combined with 200 ul of reaction mixture containing either 75 uM tyrosine, 75 uM L-Dopa, and 2 uCi L-[3,5-³H]Tyrosine in 50 mM NaPO₄ pH 6.8 (L-Dopa +), or, 75 uM tyrosine, and 2 uCi L-[3,5-³H]Tyrosine in 50 mM NaPO₄ pH 6.8 (L-Dopa -) and incubated 1 hr at 37°C. Reactions were stopped by addition of 400 ul 10% activated charcoal in 0.1N HCl and incubation on ice for 15 min. This mixture was centrifuged at 17,300 X g for 5 min, and 400 ul supernatant was then filtered through a 0.22 uM GV Durapore centifugal filter unit (Millipore) by centrifuging at 17,300 X g for 5 min. Filtrate was added to 4 ml Fisher Plus scintillation fluid and counted on a Hewlett Packard scintillation counter. Tyrosinase activity was calculated as dpm/hr/ug protein and dpm/hr/10³ cells. Each sample was analyzed with and without L-Dopa, a necessary cofactor for tyrosinase (Pomerantz, S. H., 1966, *J. Biol. Chem.* 241:161-168; McLane, *et al*., 1987, *Biochem. Biophys. Res. Commun*. 145:719-725). All reported tyrosinase values are exclusive of counts that occurred in buffer blanks and L-dopa negative aliquots. Protein was determined on aliquots of cell lysate, extracellular particulate lysate or media by the Bradford Coomassie Blue method (Bradford, 1967, *Anal. Biochem.* 72:248-254) using Bio-Rad Protein Assay Kit I.

Results (Table 9; mean ± SE) show that 3,3-dimethyl-1,2-butanediol (3,3-M-1,2-BD) and 5-norbornene-2,2-dimethanol (5-NBene-2,2-DM) result in the greatest induction of tyrosinase on both a cellular and protein basis. Although 100 uM 2,3-pyridinediol (2,3-Pyd) induced 2-fold increases of melanin (Example 3, Table 5), even 500 uM 2,3-Pyd induced only low levels of tyrosinase relative to that induced by 5 mM 5-NBene-2,2-DM or 3,3-M-1,2-BD, and, higher levels of 2,3-Pyd were toxic. 5-NBene-2,2-DM and 3,3-M-1,2-BD are nontoxic at concentrations that induce much higher levels of tyrosinase, and thus are preferred agents for induction of melanogenesis in this embodiment. Since 5-NBene-2,2-DM induces nearly equivalent levels of tyrosinase at 5-fold lower concentrations than 3,3-M-1,2-BD, it is particularly preferred. IBMX (3-isobutyl-1-methylxanthine) is well known to those in the art as potent inducer of melanogenesis and tyrosinase, and is provided as a positive control.

**Table 9**

| Sample #/Treatment | dpm/hr 10³ Cells | dpm/hr ug Protein |
|---|---|---|
| Control (n=4) | 40 ± 6 | 184 ± 27 |
| | | |
| 300 mM PG-1,2* (n=4) | 292 ± 104 | 1003 ± 370 |
| | | |
| 25 mM 3,3-M-1,2-BD* (n=2) | 1211 ± 38 | 1746 ± 220 |
| | | |
| 50 mM 1,2-cs-CPD* (n=2) | 276 ± 16 | 925 ± 53 |
| | | |
| 5 mM 5-NBene-2,2-DM (n=4) | 707 ± 54 | 1643 ± 105 |
| | | |
| 0.5 mM 2,3-Pyd* (n=2) | 142 ± 8 | 160 ± 19 |
| | | |
| 0.1 mM IBMX* (n=2) | 765 ± 53 | 2161 ± 41 |

| | | |
|---|---|---|
| *comparative compounds | | |

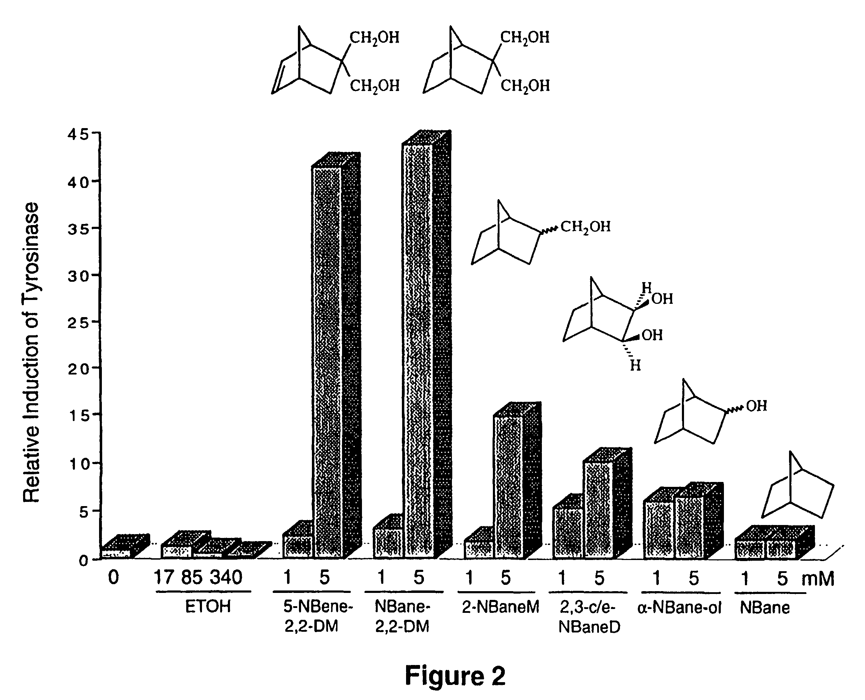

Structure activity studies with 5-NBene-2,2-DM and related compounds indicate that norbornane-2,2-dimethanol (NBane-2,2-DM) has equivalent potency for induction of tyrosinase in S91 cells (Figure 2). Thus, NBane-2,2-DM is equivalently preferred with 5-NBene-2,2-DM. Lesser induction of tyrosinase in S91 cells was induced in descending order by 2-Norbornanemethanol (2-NBaneM), 2,3-*cis*/*exo*-Norbornanediol (2,3-c/e-NBaneD), α-Norborneol (α-NBane-ol), and Norbornane (NBane). Since even NBane results in 2-fold induction of tyrosinase relative to untreated or ethanol (ETOH) treated control S91 cells, it is included as a component of this invention. In addition, since NBane induces melanogenesis, it is contemplated that all compounds containing NBane as a component of their structure may induce melanogenesis. In addition, compounds containing Norbornene (NBene) or any other unsaturated compound derived form norbornane are expected to induce melanogenesis. Thus, any saturated or unsaturated compound derived from norbornane is included as a component of this invention, including but not limited to compounds derived from borneol, camphene and camphor.

Neither the highly specific protein kinase A (PKA) inhibitor H-89 (N-[2-(p-bromocinnamylamino)-ethyl]-5-isoquinolinesulfinamide•2HCl; Chijiwa, *et al*., 1990, *J. Biol. Chem.* 265:5267-5272), nor the highly specific protein kinase C (PKC) inhibitor GF109203X (Bisindolylmaleimide; Toullec, *et al*., 1991, *J. Biol. Chem*. 266:15771-15781) inhibited induction of tyrosinase by 5-NBene-2,2-DM (Table 10). Thus, similar to results described for 1,2-propanediol in Example 3, 5-NBene-2,2-DM and related compounds are unlikely to act via activation of PKC pathways, which have been described as important for induction of melanogenesis by diacylgerols (Allan, *et al.,* 1995, *J. Invest. Dermatol*. 105:687-692; Gilchrest, *et al.,* 1996, *Photochem. Photobiol*. 63:1-10). Nor are 5-NBene-2,2-DM or related compounds likely to act via activation of PKA pathways, described as important for induction of melanogenesis by IBMX (Fuller, *et al*., 1993, *Ann. NY Acad. Sci*. 690:302-319; Fuller, *et al*., 1996, *Pigment Cell Res.* S5:65). Furthermore, addition of catalase to the cell culture media did not inhibit the action of 5-NBene-2,2-DM, indicating that unlike L-Dopa and Dopac, this and related compounds are unlikely to induce melanogenesis via generation of hydrogen peroxide or other reactive oxygen species (Karg, *et al*., 1989, *Acta Derm. Venereol*. 69: 521-524; Karg, *et al*., 1991. *J. Invest. Dermatol.* 96:224-227; Karg, *et al*., 1993, *J. Invest. Dermatol*. 100:2095-213S).

**Table 10**

| | Tyrosinase dpm/hr/ ug Protein | Relative to Control |
|---|---|---|
| Control | 398 | 1 |
| | | |
| 5 mM 5-NBene-2,2-DM | 3273 | 8.2X |
| | | |
| 1 uM H-89* | 507 | 1.3X |
| 10 uM H-89* | 1236 | 3.1X |
| | | |
| 1 uM H-89/5 mM 5-NBene-2,2-DM | 4624 | 11.6X |
| 10 uM H-89/5 mM 5-NBene-2,2-DM | 3093 | 7.8X |
| | | |
| 0.1 uM GF109203X* | 1025 | 2.6 |
| 1 uM GF109203X* | 2407 | 6.1X |
| | | |
| 0.1 uM GF109203X/5 mM 5-NBene-2,2-DM | 4679 | 11.8X |
| 1 uM GF109203X/5 mM 5-NBene-2,2-DM | 6531 | 16.4X |
| | | |
| 500 Units Catalase*/ml | 745 | 1.9X |
| 1000 Units Catalase*/ml | 691 | 1.7X |
| | | |
| 500 Units Catalase/ml/5 mM 5-NBene-2,2-DM | 2796 | 7.0X |
| 1000 Units Catalase/ml/5 mM/5-NBene-2,2-DM | 4778 | 12.0X |

| | | |
|---|---|---|
| *comparative compounds | | |

### Example 7

Tyrosinase was measured in normal human epidermal melanocytes (NHEM) using procedures identical to those described for S91 cells (Example 6), except that media from 5 day treatment periods was retained and centrifuged at 200 X g, 1600 X g, or 17,300 X g for analysis of tyrosinase activity in the extracellular exported melanosomal particulate fraction, and in the resultant supernatant media fraction. In some cases (Table 11), tyrosinase was also measured by an *in situ* assay wherein radiolabelled tyrosine was added directly to freshly replaced media of NHEM for a period of 24 hrs following a 5 day treatment period (Abdel-Malek, *et al*., 1992, *J. Cell. Physiol.* 150:416-425). Results showed that 5 mM 5-NBene-2,2-DM induced tyrosinase to a greater extent in the *in situ* assay, in cells, in extracellular particulate melanosomal fractions, and in the media of NHEM than did 25 mM 3,3-M-1,2-BD (Table 12). Both 5 mM 5-NBene-2,2-DM and 25 mM 3,3-M-1,2-BD induced more tyrosinase in each of these assays and fractions than did 1,2-PG. IBMX (3-isobutyl-1-methyl-xanthine) provided as a positive control, induced as much tyrosinase as 5 mM 5-NBene-2,2-DM in the *in situ* assay, but less in cellular, extracellular particulate and media fractions (Table 11).

**Table 11**

| Tyrosinase dpm/hr/10³ Cells | | | | | |
|---|---|---|---|---|---|
| | In Situ | Cellular | 200g Partic | 17300g* Partic | Media** |
| Control | 16.8 | 10259 | 244 | 97 | 1457 |
| 85 mM ETOH*** | 15.0 | 10201 | 442 | 132 | 1654 |
| | (1.00X) | (1.00X) | (1.00X) | (1.00X) | (1.00X) |
| | | | | | |
| 300 mM 1,2-PG*** | 16.8 | 10247 | 433 | 102 | 1864 |
| 300 mM 1,2-PG*** | 17.2 | 10875 | 923 | 241 | 2123 |
| | (1.07X) | (1.03) | (1.98X) | (1.50X) | (1.28X) |
| 25 mM | | | | | |
| 3,3-M-1,2-BD*** | 20.5 | 11728 | 1646 | 536 | 5495 |
| 25 mM | | | | | |
| 3,3-M-1,2-BD*** | 21.0 | 11730 | 2226 | 425 | 3056 |
| | (1.31X) | (1.15X) | (5.64X) | (4.20X) | (2.75X) |
| | | | | | |
| 5 mM | | | | | |
| 5-NBene-2,2-DM | 24.5 | 13838 | 6447 | 493 | 4164 |
| 5 mM | | | | | |
| 5-NBene-2,2-DM | 25.4 | 14716 | 6291 | 473 | 4639 |
| | (1.57X) | (1.40X) | (18.6X) | (4.22X) | (2.83X) |
| | | | | | |
| 0.1 mM IBMX*** | 25.3 | 10910 | 2189 | 220 | 2698 |
| 0.1 mM IBMX*** | 26.1 | 11737 | 1834 | 260 | 2935 |
| | (1.62X) | (1.11X) | (5.86X) | (2.10X) | (1.81X) |

| | | | | | |
|---|---|---|---|---|---|
| *Post 200 X g | | | | | |
| **Post 17300 X g | | | | | |
| ***comparative compounds | | | | | |

Further studies using NHEM demonstrated that, similar to results for S91 cells (Figure 2), compounds related to 5-NBene-2,2-DM may be inducers of tyrosinase (Table 12). For example, 2-norbornanemethanol (2-NBaneM) resulted in induction of tyrosinase at levels equivalent to 5-NBene-2,2-DM in NHEM both from a white adult donor and a black neonatal donor (Table 12). Thus, similar to S91 cells (Example 6), all norbornane-related compounds are contemplated to induce tyrosinase in NHEM and are thereby embodied in this invention.

**Table 12**

| *White-Adult-NHEM* | Tyrosinase dpm/hr/10³ cells | | |
|---|---|---|---|
| | In Situ | Cellular | Medial¹ |
| Control | 5.56 (1.00X) | 13992 (1.00X) | 36.3 (1.00X) |
| 1 mM 5-NBene-2,2-DM | 6.27 (1.13X) | 12740 (0.91X) | 29.9 (0.82X) |
| 5 mM 5-NBene-2,2-DM | 5.81 (1.04X) | 18467 (1.32X) | 53.1 (1.46X) |
| 1 mM 2-NBaneM* | 7.05 (1.27X) | 15257 (1.09X) | 29.2 (1.11X) |
| 5 mM 2-NBaneM* | 6.18 (1.11X) | 16077 (1.15X) | 48.1 (1.33X) |
| | | | |

| *Black-Neonatal-NHEM* | dpm/hr/10³ cells | | |
|---|---|---|---|
| | In Situ | Cellular | Media |
| Control | 12.5 (1.00X) | 9856 (1.00X) | 11.1 (1.00X) |
| 1 mM 5-NBene-2,2-DM | 13.9 (1.11X) | 10679 (1.08X) | 26.8 (2.41X) |
| 5 mM 5-NBene-2,2-DM | 14.1 (1.13X) | 15398 (1.56X) | 33.2 (2.99X) |
| 1 mM 2-NBaneM* | 12.1 (0.97X) | 10863 (1.10X) | 18.7 (1.68X) |
| 5 mM 2-NBaneM* | 12.8 (1.02X) | 17397 (1.77X) | 37.3 (3.36X) |

| | | | |
|---|---|---|---|
| ¹Unlike Table 10 where Media was from a 5 day treatment period, Media in Table 11 was from a 1 day treatment period. | | | |
| *comparative compound | | | |

### Example 8

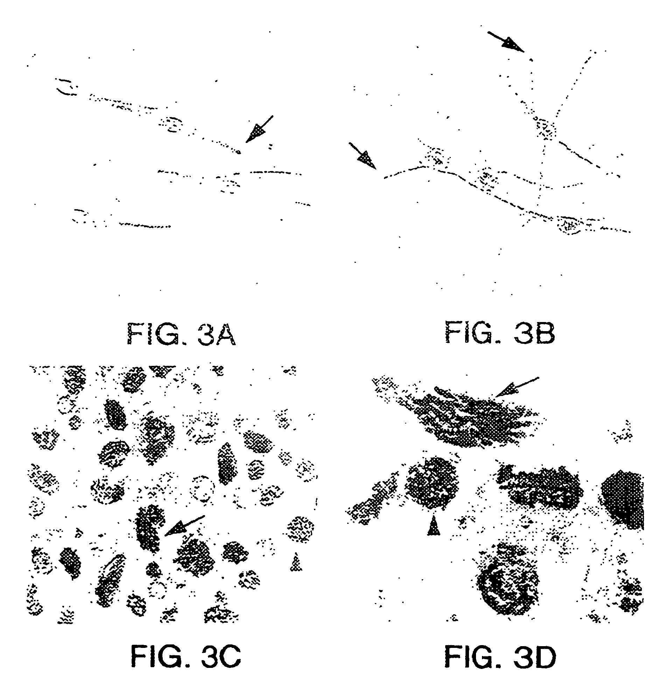

Similar to results for S91 cells treated with diols (Examples 1 and 2), treatment of normal human epidermal melanocytes (NHEM) with 5 mM 5-NBene-2,2-DM resulted in morphological changes indicative of differentiation. In the case of NHEM, induction of differentiation was marked by conversion of cells from a bipolar phenotype to a multidendritic phenotype (compare untreated NHEM in Figure 3A with 5mM 5-NBene-2,2-DM treated NHEM in Figure 3B). Additionally, the length of dendrites was increased approximately 2-3-fold following treatment with 5 mM 5-NBene-2,2-DM, and there was an increase in the number of secretatory vesicles at the termini of dendrites (arrows in Figures 3A and 3B). Electron microscopic analysis indicated that the extracellular particulate fraction secreted into the media from NHEM was comprised almost exclusively of stage III and IV melanosomes (arrows show longitudinal view and arrowheads show cross-sectional view in Figures 3C and 3D). Increased secretion of melanosomes resulting from treatment with 5 mM 5-NBene-2,2-DM was reflected in increased extracellular particulate tyrosinase activity (Example 7, Table 11).

It is well known that ultraviolet irradiation of skin results in increased dendricity of melanocytes and increased transport of melanosomes from the ends of dendritic processes to neighboring keratinocytes (Jimbow, *et al., Biology of Melanocytes*, pp. 261-289, In: Dermatology in General Medicine, eds: Fitzpatrick, *et al*., McGraw-Hill, 1994). Thus, secretion of melanosomes from melanocytes treated with 5-NBene-2,2-DM appears to parallel the physiological processes induced by sunlight in skin.

### Example 9

Highly specific inhibitors of the cAMP/PKA (protein kinase A) or PKC (protein kinase C) pathways do not inhibit induction of melanogenesis by 5-NBene-2,2-DM in S91 cells (Example 6, Table 10). However, each of the nitric oxide (NO) scavenger PTIO (2-phenyl-4,4,5,5-tetramethylimidazoline-1-oxyl-3-oxide), the cyclic guanosine monophosphate (cGMP) inhibitor LY83583 (6-anilino-5,8-quinolinequinone), and the PKG (protein kinase G) inhibitor KT58223 reduce induction of melanogenesis by 5-NBene-2,2-DM in S91 cells (Table 13). These results demonstrate that induction of melanogenesis by 5-NBene-2,2-DM occurs by the NO/cGMP/PKG pathway. Furthermore, results are similar to those obtained for ultraviolet radiation wherein induction of melanogenesis did not occur via either the cAMP/PKA or PKC pathways (Friedmann and Gilchrest, 1987, *J. Cell. Physiol* 133:88-94; Carsberg, *et al., J. Cell. Sci.* 107:2591-2597), but rather occurred via the NO/cGMP/PKG pathway (Romero-Graillet, *et al*., 1996, *J. Biol. Chem*. 271:28052-28056; Romero-Graillet, *et al*., 1997, *J. Clin. Invest*. 99:635-642). Moreover, unlike IBMX (3-isobutyl-1-methylxanthine) and MSH (melanocyte stimulating hormone) which induce melanogenesis by the cAMP/PKA pathway (Wintzen and Gilchrest, 1996, *J. Invest. Dermatol*. 106:3-10; Fuller, *et al*., 1993, *Ann. NY Acad. Sci*. 690:302-319), and DAG (diacylglycerol) which induces melanogenesis by the PKC pathway (Allan, *et al*., 1995, *J. Invest. Dermatol*. 105:687-692), 5-NBene-2,2-DM induces melanogenesis by the NO/cGMP/PKG pathway similar to ultraviolet radiation.

**Table 13**

| NO/PKG Inhibitors - Experiment 1 | | |
|---|---|---|
| | dpm/hr/ 10³ cells | % of 5-NBene-2,2-DM |
| Induction | | |
| 5 mM 5-NBene-2,2-DM (n=4) | 5018 ± 415¹ | 100% |
| | | |
| 5 mM 5-NBene-2,2-DM/20 uM PTIO² (n=2) | 3703 ± 262 | 74% |
| | | |
| 5 mM 5-NBene-2,2-DM/0.5 uM KT5823³ (n=2) | 1528 ± 190 | 31% |
| | | |

| NO/PKG Inhibitors - Experiment 2 | | |
|---|---|---|
| | dpm/hr/ 10³ cells | % of 5-NBene-2,2-DM |
| Induction | | |
| 5 mM 5-NBene-2,2-DM (n=4) | 5640 ± 323 | 100% |
| | | |
| 5 mM 5-NBene-2,2-DM/20 uM PTIO² (n=2) | 4078 ± 429 | 72% |
| | | |
| 5 mM 5-NBene-2,2-DM/40 uM PTIO (n=2) | 3351 ± 994 | 59% |
| | | |
| 5 mM 5-NBene-2,2-DM/0.5 uM KT5823³ (n=2) | 2940 ± 261 | 52% |
| | | |
| 5 mM 5-NBene-2,2-DM/1.0 uM KT5823 (n=2) | 1688 ± 324 | 30% |
| | | |
| | | |

| cGMP Inhibitor - Experiment 3 | | |
|---|---|---|
| | dpm/hr/ 10³ cells | % of 5-NBene-2,2-DM |
| Induction | | |
| 5 mM 5-NBene-2,2-DM (n=4) | 6388 ± 460¹ | 100% |
| | | |
| 5 mM 5-NBene-2,2-DM/0.1 uM LY83583 (n=2) | 1389 ± 64 | 22% |
| | | |
| 5 mM 5-NBene-2,2-DM/0.2 uM LY83583 (n=2) | 300 ± 84 | 5% |

| | | |
|---|---|---|
| ¹X ± SE | | |
| ²PTIO: Nitric oxide scavenger | | |
| ³KT5823: PKG inhibitor | | |

### Example 10

The PC12 rat pheochromocytoma cell line was obtained from American Type Culture Collection (ATCC). Cells were cultured in 85% RPMI 1640 medium, 10% horse serum (heat inactivated at 56°C for 30 minutes, 5% fetal bovine serum, 25 U/ml penicillin, and 25 ug/ml streptomycin (Greene, *et al*., 1991, "Methodologies for the culture and experimental use of the rat PC12 rat pheochromocytoma cells line", pp. 207-225, In: Culturing Nerve Cells, The MIT Press, Cambridge, Massachusetts). Cells were cultured directly on plastic dishes at 37°C in 5% CO₂ in a humidified incubator.

PC12 rat pheochromocytoma cells are considered to be an excellent model for neuronal cells because they respond to treatment with nerve growth factor (NGF) by acquisition of a number of properties of neurons including cessation of proliferation, extension of neurons, acquisition of electrical excitability, and increased neurotransmitter synthesis (Greene, *et al*., 1991 and references therein). In addition, PC12 cells are used as a model for studies of prevention or cure of neurodegenerative diseases since they provide a robust screen for agents that maintain neuron survival and prevent neuron cell death in serum-free media (Rukenstein, *et al*., 1991, *J. Neurosci*. 11:255-2563). Agents are considered to be potentially useful for treatment of neurodegenerative disorders if they not only promote PC12 cell survival, but also increase neurite outgrowth (Rukenstein, *et al*., 1991). Agents are considered to be particularly useful for treatment of neurodegenerative disorders if they promote PC12 cell survival and neurite outgrowth in the absence of "priming" with NGF (Rukenstein, *et al*., 1991). By virtue of their ability to express tyrosine hydroxylase and thereby synthesize dopamine, PC12 cells are considered to be an especially good model for studies of Parkinson's disease (Michel, *et al*., 1994, *Europ. J. Neurosci. Assoc.* 6:577-586 and references therein). In addition, neurite outgrowth in PC12 cells has been used to identify agents that stimulate the regeneration of severed neuronal axons in the peripheral nerves of adult mammals (Sandrock, A. W. and Matthew, W. D., 1987, *Proc. Natl. Acad. Sci. U.S.A.* 84:6934-6938). Moreover, PC12 cells have been used as a model to study aspects of Alzheimer's disease (Shen, *et al*., 1995, *Brain Res.* 671:282-292), amyotrophic lateral sclerosis (Durham, *et al*., 1995, *Clin. Exp. Pharmacol. Physiol*. 22:366-67), Down's syndrome (Groner, *et al*., 1994, *Biomed. Pharmacother*. 48:231-240), and age-related neurodegeneration (Taglialatela, *et al*., 1996, *J. Neurochem*. 66:1826-1835).

For testing compounds for induction of dendricity (neurite outgrowth) and tyrosine hydroxylase activity in this invention, cells were plated at 15,000 cells/35 mm dish. Two days following plating, cell culture media was replaced with that containing treatments. One week later, media and treatments were replaced with fresh media and treatments. Two weeks following the initial treatments, cells were examined microscopically, and the portion of cells exhibiting dendricity was estimated. Cells were harvested by trypsinization and counted by Coulter Counter. Cells were pelleted by centrifugation at 200 X g, and cell pellets were lysed in 600 ul 50 mM Tris/Acetate pH 6.0/0.2% Triton X-100 by vortexing, sonicating 5 seconds, incubating on ice for 30 minutes, followed by revortexing. Protein was determined on aliquots of cell lysate by the Bradford Coomassie Blue method (Bradford, 1967, *Anal. Biochem.* 72:248-254) using Bio-Rad Protein Assay Kit I. Tyrosine hydroxylase activity was determined by incubating 100 ul of PC12 cell lysate with 100 ul of the following reaction mixture at 37°C for 15 min: 200 mM sodium acetate pH 6.0, 50 uM tyrosine, 2000 U Cat/ml, 50 mU dihydropteridine reductase/ml, 0.1 mM NADH final, 200,000 cpm 3H tyrosine/100 ul, 0.1 mM NSD1015 (3-hydroxybenzylhydrazine), and 100 uM tetrahydrobiopterin (BH4) (Nagatsu, *et al*., 1969, *Anal. Biochem.* 9:122-126; Ribeiro, *et al*. 1991, *J. Biol. Chem.* 16207-16211). Reactions were stopped by addition of 200 ul 10% activated charcoal in 0.1N HCl and incubation on ice for 15 min. This mixture was centrifuged at 17,300 X g for 5 min, and 200 ul supernatant was then filtered through a 0.22 uM GV Durapore centrifugal filter unit (Millipore) by centrifuging at 17,300 X g for 5 min. Filtrate was added to 4 ml Fisher Plus scintillation fluid and counted on a Hewlett Packard scintillation counter. Tyrosine hydroxylase activity was measured as tritium release and was calculated as dpm/ug protein and dpm/10³ cells per hour.

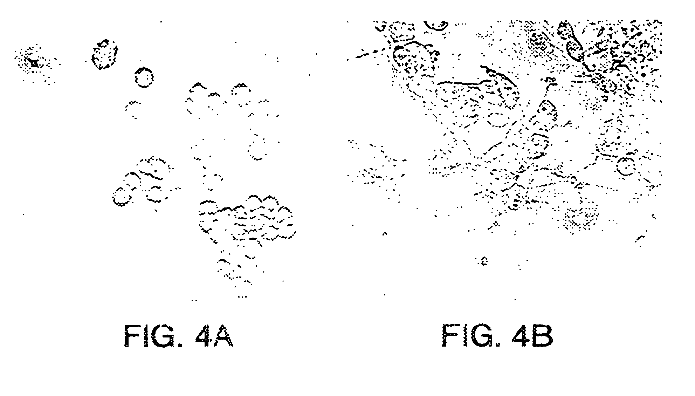

Microscopic examination showed that a large portion of PC12 cells treated with 5 mM 5-norbornene-2,2-dimethanol (5-NBene-2,2-DM) acquired dendritic processes (Table 14, and compare untreated PC12 cells in Figure 4A with 5-NBene-2,2-DM treated PC12 cells in Figure 4B). Lesser increases of dendritic processes were noted following treatment with 3,3-dimethyl-1,2-butandiol (3,3-M-1,2-BD) or 1,2-propylene glycol (1,2-PG) (Table 14). The most notable increases of tyrosine hydroxylase activity resulted from treatment with 25 mM 3,3-M-1,2-BD and 5 mM 5-NBene-2,2-DM (Table 14). Treatment with 1,2-PG, 3,3-M-1,2-BD and 5-NBene-2,2-DM increased the amount of protein per cells, a feature often associated with induction of differentiation. Increases of protein per cells were manifested morphologically as an increase in cell size (compare untreated PC12 cells in Figure 4A with 5-NBene-2,2-DM treated PC12 cells in Figure 4B). Examination of the data in Table 14 shows that increases of tyrosine hydroxylase per cell as a result of treatment with 1,2-PG, 3,3-M-1,2-BD or 5-NBene-2,2-DM, were in part, a result of increases of the amount of protein per cell. Ethanol (ETOH), used as a solvent for 3,3-M-1,2-BD and 5-NBene-2,2-DM, and IBMX (3-isobutly-1-methylxanthine), which increases cellular cAMP levels, resulted in only minor effects relative to the agents of this invention.

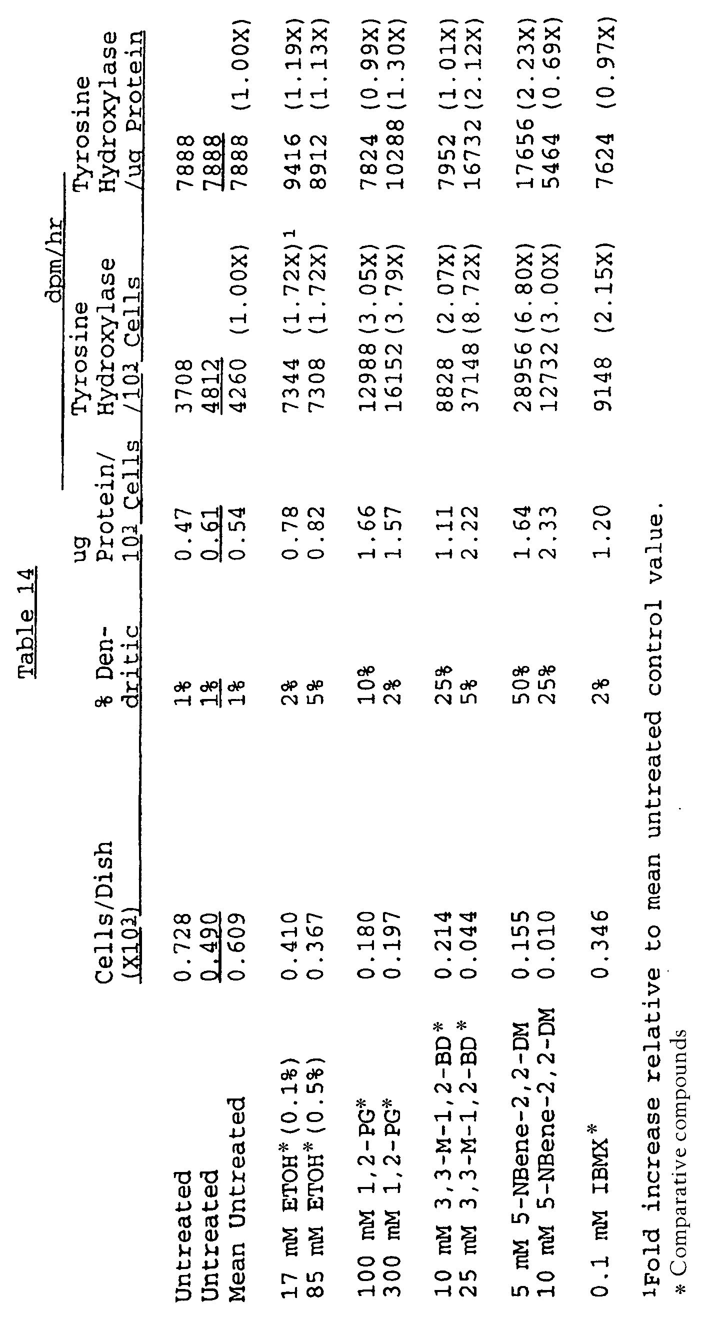

The reduced cell numbers resulting from treatment with 1,2-PG, 3,3-M-1,2-BD or 5-NBene-2,2-DM are in part indicative of the differentiation process induced by treatments. However, in the case of treatment with 25 mM 3,3-M-1,2-BD and 10 mM 5-NBene-2,2-DM, some cells detached concomitantly with the acquisition of dendricity that occurred earlier than for other treatments. This detachment phenomenon has been noticed previously for PC12 cells induced to differentiate with NGF, and can be avoided by coating treatment dishes with collagen (reviewed in Greene, *et al*., 1991). Treatment with collagen also shortens the time required for dendrite formation and greatly increases the extent of dendrite formation in response to treatment with NGF (reviewed in Greene, *et al*., 1991). Thus, it is contemplated that the compounds of this invention will prove to exhibit more activity when tested on collagen-coated dishes.

Induction of differentiation as indicated by induction of dendricity, induction of tyrosine hydroxylase activity, increased cellular protein levels and induction of cell cycle arrest as indicated by reduced growth, indicate that the compounds of this invention can act as chemotherapeutic agents for treatment of neural tumorous and cancerous disorders and additional neural proliferative disorders. In addition, it is contemplated that the compounds of this invention will treat tumorous, cancerous and proliferative disorders arising from additional cell types.

It should be particularly noted that the compounds of this invention induced dendricity and tyrosine hydroxylase activity in the absence of priming with NGF, a prerequisite for induction of neurite extension by many other agents tested on PC12 cells (Steiner, *et al*. 1997, *Nature Medicine* 3:421-428; Rukenstein, *et al*. 1991, *J. Neurosci*. 11:2552-2563). Several agents under consideration as treatments for neurodegenerative diseases do not promote neurite extension even in NGF-primed PC12 cells (*e.g*., IGF-I and IGF-II; Rukenstein, *et al*., 1991 and references therein). Moreover, many agents under consideration for treatment of neurodegenerative diseases including GDNF (glial cell-derived neurotrophic factor) being developed for treatment of Parkinson's disease are neurotrophic peptides that cannot cross the blood-brain barrier and therefore require gene therapy implantation at the site of action (Haase, *et al*. 1997, *Nature Medicine* 3:429-436). Furthermore, L-Dopa which is presently used for treatment of Parkinson's disease is toxic (Yahr, M. D. 1993, *Adv. Neurol*. 60:11-17), in part, by generation of peripherally formed dopamine (Riederer, *et al*. 1993, *Adv. Neurol*. 60:626-635), and in part, by virtue of its ability to form highly reactive semiquinone and quinones via autooxidation (Karg, *et al*. 1989, *Acta Derm. Venereol*. 69:521-524). Given that the agents of the present invention: (i) act directly without a requirement for NGF; (ii) induce neuronal differentiation thereby setting into motion cellular reprogramming to the desired phenotype; (iii) induce tyrosine hydroxylase, the rate-limiting enzyme in dopamine synthesis; (iv) are small molecule drugs that are likely to cross the blood brain barrier; and (v) have no known ability to form semiquinone, quinone or other toxic intermediates, it is contemplated that the agents of this invention will be particularly advantageous for treatment of neurodegenerative diseases including but not limited to Parkinson's disease.

### Example 11

Cloudman S91 mouse melanoma cells were obtained from ATCC and cultured in MEM (BioWhittaker) with 10% calf serum (BioWhittaker or Hyclone). Cells were plated at 10⁵ cells/well in 6-well plates the day before treatments, in media containing 10% calf serum. Media was changed to MEM with 2% calf serum concomitant with addition of treatments (Eller, *et al*., 1996, *Proc. Natl. Acad. Sci.* 93:1087-1092). Six days later, media was removed and S91 cells were washed twice with 2 ml 1XPBS (BioWhittaker), 1 ml of 0.05% trypsin/EDTA (BioWhittaker) was added, and cells were incubated at 37°C until detached from plastic dishes. Four ml of media containing 10% calf serum was added, cells were mixed by pipette action until no clumps of cells remained, and 0.5 ml were counted on a Coulter counter.

Tyrosinase was analyzed using previously described procedures (Pomerantz, 1966, *J. Biol. Chem*. 241:161-168). Cells were solubilized by sonicating for 5 seconds in 600 ul 50 mM phosphate buffer pH 6.8 containing 0.5% Triton-X100, followed by vortexing, incubation on ice for 30 minutes, and then revortexing. From this, 200 ul aliquots were combined with 200 ul of reaction mixture containing either 75 uM tyrosine, 75 uM L-Dopa, and 2 uCi L-[3,5-³H]Tyrosine in 50 mM NaPO₄ pH 6.8 (L-Dopa +), or, 75 uM tyrosine, and 2 uCi L-[3,5-³H]Tyrosine in 50 mM NaPO₄ pH 6.8 (L-Dopa -), and then incubated 1 hr at 37°C. Reactions were stopped by addition of 400 ul 10% activated charcoal in 0.1N HCl and incubation on ice for 15 min. This mixture was centrifuged at 17,300 X g for 5 min, and 400 ul supernatant was then filtered through a 0.22 uM GV Durapore centrifugal filter unit (Millipore) by centrifuging at 17,300 X g for 5 min. Filtrate was added to 4 ml Fisher Plus scintillation fluid and counted on a Hewlett Packard 2000A scintillation counter. Tyrosinase activity was calculated as dpm/10³ cells. Each sample was analyzed with and without L-Dopa, a necessary cofactor for tyrosinase (Pomerantz, 1966). All reported tyrosinase values are exclusive of counts that occurred in buffer blanks and L-dopa negative aliquots.

It has been previously demonstrated that a variety of aliphatic and alicyclic diols including 5-norbornene-2,2-dimethanol (5-NBene-2,2-DM) induce melanogenesis in S91 cells. The results presented in Table 15 show that induction of tyrosinase (the rate-limiting enzyme in melanogenesis) by 5-NBene-2,2-DM is not blocked by highly specific inhibitors of the PKC and PKA pathways. In fact, treatment of S91 cells with either the highly specific PKA inhibitor H-89 (Chijiwa, *et al*., 1990, *J. Biol. Chem.* 265:5267-5272), or the highly specific PKC inhibitor GF109203X (Toullec, *et al*., 1991, *J. Biol. Chem*. 266:15771-15781) resulted in augmentation of basal and 5-NBene-2,2-DM-induced tyrosinase levels (Table 15). Thus, 5-NBene-2,2-DM does not appear to act via either the PKC or PKA pathways.

In contrast, both the nitric oxide (NO) scavenger PTIO (2-phenyl-4,4,5,5-tetramethylimidazoline-1-oxyl-3-oxide), the cyclic guanosine monophosphate (cGMP) inhibitor LY83583 (6-anilino-5,8-quinolinequinone), and the PKG (cGMP-activated protein kinase) inhibitor KT5823 reduced induction of melanogenesis by 5-NBene-2,2-DM in S91 cells (Table 16). These results demonstrate that induction of melanogenesis by 5-NBene-2,2-DM occurs by the NO/cGMP/PKG pathway.

Previously, it has been demonstrated that NO donors can stimulate melanogenesis in normal human melanocytes (Romero-Graillet, *et al*., 1996, *J. Biol. Chem*. 271). Results presented here demonstrate that 5-NBene-2,2-DM can stimulate melanogenesis with a potency equivalent or greater than that of NO donors, even though 5-NBene-2,2-DM has no ability to donate NO. Since induction of melanogenesis by 5-NBene-2,2-DM occurs by the NO/cGMP/PKG pathway, 5-NBene-2,2-DM must directly stimulate NO synthesis.

These results demonstrate that stimulation of NO synthesis and the cGMP/PKG pathway by 5-NBene-2,2-DM provides an efficient alternative to stimulation of this pathway by NO donors. Thus, 5-NBene-2,2-DM and related compounds described in this invention will serve as alternative therapeutics for treatment of a variety of diseases mediated by perturbations of the NO/cGMP/PKG pathway.

**Table 15**

| PKA/PKC Inhibitors | | |
|---|---|---|
| | dpm/hr/ 10³ cells | % of 5-NBene-DM Induction |
| 5 mM 5-NBene-2,2-DM (n=2) | 2142 ± 185¹ | 100% |
| | | |
| 5 mM 5-NBene-2,2-DM/1 uM H-89² (n=1) | 3255 | 152% |
| 5 mM 5-NBene-2,2-DM/10 uM H-89 (n=1) | 2428 | 113% |
| | | |
| 5 mM 5-NBene-2,2-DM/0.1 uM GF109203X³ (n=1) | 2700 | 126% |
| 5 mM 5-NBene-2,2-DM/1.0 uM GF109203X (n=1) | 5055 | 236% |
| | | |

| | dpm/hr/ 10³ cells | % of Control |
|---|---|---|
| Untreated Control (n=2) | 128 ± 4 | 100% |
| | | |
| 1 uM H-89² (n=1) | 177 | 138% |
| 10 uM H-89 (n=1) | 765 | 598% |
| | | |
| 0.1 uM GF109203X³(n=1) | 270 | 211% |
| 1.0 uM GF109203X (n=1) | 650 | 508% |

| | | |
|---|---|---|
| ¹X ± SE | | |
| ²H-89: PKA inhibitor | | |
| ³GF109203X: PKC inhibitor | | |

**Table 16**

| NO/PKG Inhibitors - Experiment 1 | | |
|---|---|---|
| | dpm/hr/ 10³ cells | % of 5-NBene-2,2-DM Induction |
| 5 mM 5-NBene-2,2-DM (n=4) | 5018 ± 415¹ | 100% |
| | | |
| 5 mM 5-NBene-2,2-DM/20 uM PTIO⁴ (n=2) | 3703 ± 262 | 74% |
| | | |
| 5 mM 5-NBene-2,2-DM/0.5 uM KT5823⁵ (n=2) | 1528 ± 190 | 31% |
| | | |

| NO/PKG Inhibitors - Experiment 2 | | |
|---|---|---|
| | dpm/hr/ 10³ cells | % of 5-NBene-2,2-DM Induction |
| 5 mM 5-NBene-2,2-DM (n=4) | 5640 ± 323 | 100% |
| | | |
| 5 mM 5-NBene-2,2-DM/ 20 uM PTIO⁴ (n=2) | 4078 ± 429 | 72% |
| 5 mM 5-NBene-2,2-DM/ 40 uM PTIO (n=2) | 3351 ± 994 | 59% |
| | | |
| 5 mM 5-NBene-2,2-DM/ 0.5 uM KT5823⁵ (n=2) | 2940 ± 261 | 52% |
| 5 mM 5-NBene-2,2-DM/1.0 uM KT5823 (n=2) | 1688 ± 324 | 30% |
| | | |

| cGMP Inhibitor - Experiment 3 | | |
|---|---|---|
| | dpm/hr/ 10³ cells | % of 5-NBene-2,2-DM Induction |
| 5 mM 5-NBene-2,2-DM (n=4) | 6388 ± 460¹ | 100% |
| | | |
| 5 mM 5-NBene-2,2-DM/0.1 uM LY83583⁶ (n=2) | 1389 ± 64 | 22% |
| 5 mM 5-NBene-2,2-DM/0.2 uM LY83583 (n=2) | 300 ± 84 | 5% |

| | | |
|---|---|---|
| ¹X ± SE | | |
| ⁴PTIO: Nitric oxide scavenger | | |
| ⁵KT5823: PKG inhibitor | | |
| ⁶LY85583: cGMP inhibitor | | |

## Claims

1. Use of one or more compounds as defined below in the preparation of a medicament for the treatment, prevention, or alteration of a disease or condition mediated by deficiencies in the NO/cGMP/PKG signal transduction pathway, which is selected from hypopigmentation disorders; skin proliferative disorders or disorders of keratinization; neurodegenative disorders; heart disease, including stable angina pectoris, unstable angina, myocardial infarction, myocardial failure associated with myocardial ischemia, atherosclerosis, vascular hypertrophy and thrombosis; hypertension; stroke; primary pulmonary hypertension, chronic obstructive pulmonary disease and adult respiratory distress syndrome; microvascular functional abnormalities in diabetes; hemostatic irregularities of glomerular vascular and tubular function; disorders of bladder function and reflex relaxation for micturition; disorders of neurotransmitter release, neuron morphogenesis, synaptic plasticity and neuroendocrine regulation; regional pain, including migraine headaches; benign anal disease; impotence; inhibition of tumor growth; and stimulation of wound healing:
(i) diols having the structure: 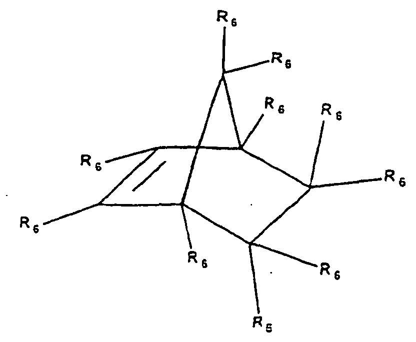
(ii) diols having the structure: 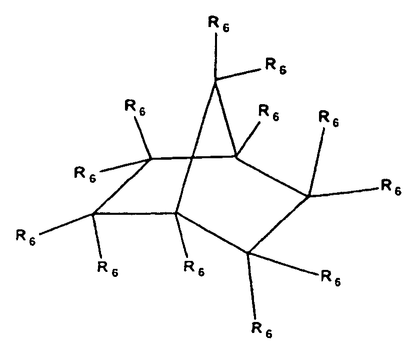
(iii) pharmaceutically-acceptable salts of (i); and
(iv) pharmaceutically-acceptable salts of (ii); wherein:
each R₆ is independently selected from hydrogen; halogen; or a group containing from one atom to twenty atoms, one of which is carbon, nitrogen, oxygen, or sulfur; hydroxyl, hydroxymethyl, - (CH₂)ₙOH, -(CH₂)ₙOR₁, -(CH₂)ₙ-CH(OH)-CHOH, - (CH₂)ₙ-CH(OH)-CH(OH)R₁, -(CH₂)ₙ-CH(OH)-(CH₂)ₙ-CH₂(OH), - (CH₂)ₙ-CH(OH)-(CH₂)ₙ-CH(OH)R₁ or -CH₂OR₃, wherein each n is independently O or an integer of from 1 to 25;
each R₁ is independently selected from hydrogen; halogen; or a group containing from one atom to twenty atoms, one of which is carbon, nitrogen, oxygen, or sulfur;
and
each R₃ is independently selected from hydrogen or an acyl or amino acyl group containing from one atom to twenty atoms, at least one of which is carbon, nitrogen, oxygen, or sulfur.

2. A use as claimed in claim 1 wherein the one or more compounds is/are selected from:
2-(propyl-1,2-diol)-norbornane, 5-norbornene-2,2-dimethanol, norbornane-2,2-dimethanol, 2,3-norbornanediol (exo or endo or cis or trans), 2,3-cis-exo-norbornanediol, and 2-endo-hexadecylamino-5-norbornene-2,3-exo-dimethanol.

3. A use as claimed in claim 1 wherein the one or more compounds is/are selected from:
2-(propyl-1,2-diol)-norbornane, 5-norbornene-2,2-dimethanol; norbornane-2,2-dimethanol; and 2,3-cis-exo-norbornanediol.

4. A use as claimed in any of claims 1 to 3 wherein a suitable carrier is also present.

5. A use as claimed in any of claims 1 to 4 wherein the disease or condition is selected from heart disease, including stable angina pectoris, unstable angina, myocardial infarction, myocardial failure associated with myocardial ischemia, atherosclerosis, vascular hypertrophy and thrombosis; hypertension; stroke; primary pulmonary hypertension, chronic obstructive pulmonary disease and adult respiratory distress syndrome; microvascular functional abnormalities in diabetes; impotence; stimulation of wound healing; and hypopigmentation disorders.

6. A use as claimed in any of claims 1 to 4 wherein the disease or condition is selected from disorders of neurotransmitter release, neuron morphogenesis, synaptic plasticity and neuroendocrine regulation; regional pain, including migraine headaches; inhibition of tumor growth; skin proliferative disorders or disorders of keratinisation; and neurodegenerative disorders.

7. A use as claimed in any of claims 1 to 4 wherein the disease or condition is selected from hemostatic irregularities of glomerular vascular and tubular function; disorders of bladder function and reflex relaxation for micturition; and benign anal disease.

## Patentansprüche

1. Verwendung einer oder mehrerer Verbindung wie unten definiert in der Herstellung eines Medikamentes zur Behandlung, Vorbeugung oder Veränderung einer Erkrankung oder eines Zustandes, vermittelt durch Mängel im NO/cGMP/PKG Signaltransduktionsweg, die aus Hypopigmentierungsstörungen, proliferativen Hautstörungen oder Störungen der Keratinisierung, neurodegenerativen Störungen, Herzkrankheit, einschließlich stabiler Angina Pectoris, instabiler Angina, Herzinfarkt, mit Herzischämie verbundenem Herzversagen, Atheriosklerose, vaskulärer Hypertrophie und Thrombose, Hypertension; Schlaganfall; primärer pulmonarer Hypertension, chronisch obstruktiver Lungenkrankheit, Schocklunge, mikrovaskulären funktionellen Abnormalitäten bei Diabetes; hämostatischen Irregularitäten der glomerulären, vaskulären und tubulären Funktion; Störungen der Blasenfunktion und der Reflexrelaxation zur Blasenentleerung; Störungen der Neurotransmitterfreisetzung, Neuron-Morphogenese, synaptischer Plastizität und neuroendokriner Regulation; regionalem Schmerz, einschließlich Migränekopfschmerz; benigner Analerkrankung; Impotenz; Hemmung des Tumorwachstums; und Stimulierung der Wundheilung ausgewählt ist:
(i) Diole mit der Struktur: 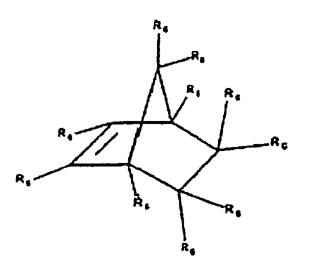
(ii) Diole mit der Struktur: 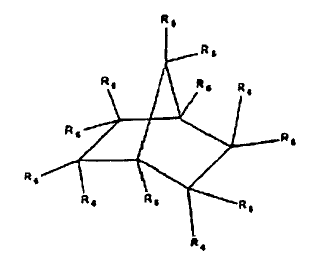
(iii) pharmazeutisch akzeptable Salze von (i);
und
(iv) pharmazeutisch akzeptable Salze von (ii);
wobei:
jedes R₆ unabhängig aus Wasserstoff; Halogen oder einer Gruppe, die von 1 Atom bis 20 Atome enthält, von denen eines Kohlenstoff, Stickstoff, Sauerstoff oder Schwefel ist; Hydroxyl, Hydroxymethyl,
-(CH₂)ₙOH, -(CH₂)ₙOR₁, -(CH₂)ₙ-CH(OH)-CHOH,
-(CH₂)ₙ-CH(OH)-CH(OH)R₁, -(CH₂)ₙ-CH(OH)-(CH₂)ₙ-CH₂(OH),
-(CH₂)ₙ-CH(OH)-(CH₂)ₙ-CH(OH)R₁ oder -CH₂OR₃ ausgewählt ist, wobei jedes n unabhängig O oder eine ganze Zahl von 1 bis 25 ist;
jedes R₁ unabhängig aus Wasserstoff; Halogen; oder eine Gruppe ausgewählt ist, die von einem Atom bis 20 Atome enthält, von denen eines Kohlenstoff, Stickstoff, Sauerstoff oder Schwefel ist; und
jedes R₃ unabhängig aus Wasserstoff oder einer Acyl- oder Aminoacylgruppe ausgewählt ist, die von 1 Atom bis 20 Atome enthält, von denen zumindest eines Kohlenstoff, Stickstoff, Sauerstoff oder Schwefel ist.

2. Verwendung nach Anspruch 1, wobei die ein oder mehreren Verbindungen aus folgendem ausgewählt ist/sind:
2-(Propyl-1,2-diol)-norbornan, 2-Norbornen-2,2-dimethanol, Norbornan-2,2-dimethanol, 2,3-Norbomandiol (exo oder endo oder cis oder trans), 2,3-cis-exo-Norbornandiol und 2-endo-Hexadecylamino-5-norbonen-2,3-exo-dimethanol.

3. Verwendung nach Anspruch 1, wobei die ein oder mehreren Verbindungen aus folgendem ausgewählt ist/sind:
2-(Propyl-1,2-diol)-norbornan, 5-Norbornen-2,2-dimethanol; Norbornan-2,2-dimethanol; und 2,3-cis-exo-Norbornandiol.

4. Verwendung nach einem der Ansprüche 1 bis 3, wobei auch ein geeigneter Träger vorhanden ist.

5. Verwendung nach einem der Ansprüche 1 bis 4, wobei die Erkrankung oder der Zustand aus Herzkrankheit, einschließlich stabiler Angina Pectoris, instabiler Angina, Herzinfarkt, mit Herzischämie verbundenem Herzversagen, Atheriosklerose, vaskulärer Hypertrophie und Thrombose, Hypertension; Schlaganfall; primärer pulmonarer Hypertension, chronisch obstruktiver Lungenkrankheit und Schocklunge; mikrovaskulären funktionellen Abnormalitäten bei Diabetes; Impotenz; Stimulierung der Wundheilung; und Hypopigmentierungsstörungen ausgewählt ist.

6. Verwendung nach einem der Ansprüche 1 bis 4, wobei die Erkrankung oder der Zustand aus Störungen der Neurotransmitterfreisetzung, Neuron-Morphogenese, synaptischer Plastizität und neuroendokriner Regulation; regionalem Schmerz, einschließlich Migränekopfschmerz; Hemmung des Tumorwachstums; proliferativen Hautstörungen oder Störungen der Keratinisierung, neurodegenerativen Störungen ausgewählt ist.

7. Verwendung nach einem der Ansprüche 1 bis 4, wobei die Erkrankung oder der Zustand aus hämostatischen Irregularitäten der glomerulären, vaskulären und tubulären Funktion; Störungen der Blasenfunktion und der Reflexrelaxation zur Blasenentleerung; und benigner Analerkrankung ausgewählt ist.

## Revendications

1. Utilisation d'un ou plusieurs composés comme définis ci-après dans la préparation d'un médicament pour le traitement, la prévention ou l'altération d'une maladie ou d'un état pathologique dû à des déficiences de la voie de transduction du signal NO/GMPc/PKG, qui est sélectionné parmi les anomalies de la pigmentation ; les maladies prolifératives de la peau ou les anomalies de la kératinisation ; les maladies neurodégénératives ; les cardiopathies, y compris l'angine de poitrine stable, l'angine instable, l'infarctus du myocarde, l'insuffisance myocardique associée à une ischémie myocardique, l'athérosclérose, l'hypertrophie et la thrombose vasculaire ; l'hypertension ; les accidents vasculaires cérébraux ; l'hypertension pulmonaire primaire, la bronchopneumopathie chronique obstructive et le syndrome de détresse respiratoire de l'adulte ; les anomalies microvasculaires fonctionnelles associées au diabète ; les anomalies hémostatiques de la fonction glomérulaire, vasculaire et tubulaire ; les troubles de la fonction vésicale et de la relaxation réflexe pour la miction ; les troubles de la libération des neurotransmetteurs, la morphogenèse neuronale, la plasticité synaptique et la régulation neuroendocrinienne ; les douleurs locales, y compris les migraines ; les maladies anales bénignes ; l'impuissance ; l'inhibition de la croissance des tumeurs ; et la stimulation de la cicatrisation :
(i) les diols ayant la structure : 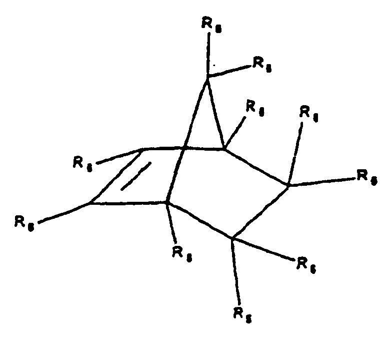
(ii) les diols ayant la structure : 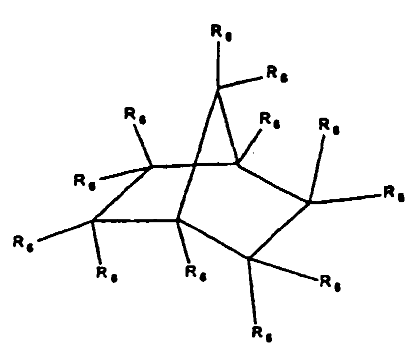
(iii) les sels pharmaceutiquement acceptables de (i) ; et
(iv) les sels pharmaceutiquement acceptables de (ii) ; et
dans lesquelles
chaque R₆ est indépendamment choisi parmi un atome d'hydrogène, un atome d'halogène ou un groupe contenant d'un atome à vingt atomes, dont l'un est un atome de carbone, d'azote, d'oxygène ou de soufre ; un groupe hydroxyle, hydroxyméthyle, -(CH₂)ₙOH, -(CH₂)ₙOR₁, -(CH₂)ₙ-CH(OH)-CHOH, -(CH2) n-CH (OH)-CH(OH)R₁, -(CH₂)ₙ-CH(OH)-(CH₂)ₙ-CH₂(OH), -(CH₂)ₙ-CH(OH)-(CH₂)ₙ-CH(OH)R₁, ou -CH₂OR₃, dans lesquels chaque n vaut indépendamment 0 ou un entier de 1 à 25 ;
chaque R₁ est indépendamment choisi parmi un atome d'hydrogène, un atome d'halogène ou un groupe contenant de un atome à vingt atomes, dont l'un est un atome de carbone, d'azote, d'oxygène ou de soufre ;
et
chaque R₃ est indépendamment choisi parmi un atome d'hydrogène ou un groupe acyle ou amino-acyle contenant de un atome à vingt atomes, dont au moins un est un atome de carbone, d'azote, d'oxygène ou de soufre.

2. Utilisation selon la revendication 1, dans laquelle l'un ou les plusieurs composés sont choisis parmi :
le 2-(propyl-1,2-diol)-norbornane, le 5-norbornène-2,2-diméthanol, le norbornane-2,2-diméthanol, 2,3-norbornanediol (exo ou endo, ou cis ou trans), le 2,3-cis-exo-norbornanediol et le 2-endo-hexadécylamino-5-norbornène-2,3-exo-diméthanol.

3. Utilisation selon la revendication 1, dans laquelle l'un ou les plusieurs composés sont choisis parmi :
le 2-(propyl-1,2-diol)-norbornane, le 5-norbornène-2,2-diméthanol, le norbornane-2,2-diméthanol et le 2,3-cis-exo-norbornanediol.

4. Utilisation selon l'une quelconque des revendications 1 à 3, dans laquelle un véhicule adapté est également présent.

5. Utilisation selon l'une quelconque des revendications 1 à 4, dans laquelle la maladie ou l'état pathologique est choisi parmi les cardiopathies, y compris l'angine de poitrine stable, l'angine instable, l'infarctus du myocarde, l'insuffisance myocardique associée à une ischémie myocardique, l'athérosclérose, l'hypertrophie et la thrombose vasculaire ; l'hypertension ; les accidents vasculaires cérébraux ; l'hypertension pulmonaire primaire, la bronchopneumopathie chronique obstructive et le syndrome de détresse respiratoire de l'adulte ; les anomalies microvasculaires fonctionnelles associées au diabète ; l'impuissance ; la stimulation de la cicatrisation ; et les anomalies de la pigmentation.

6. Utilisation selon l'une quelconque des revendications 1 à 4, dans laquelle la maladie ou l'état pathologique est choisi parmi les troubles de la libération des neurotransmetteurs, la morphogenèse neuronale, la plasticité synaptique et la régulation neuroendocrinienne ; les douleurs locales, y compris les migraines ; l'inhibition de la croissance des tumeurs ; les maladies prolifératives de la peau ou les anomalies de la kératinisation ; et les maladies neurodégénératives.

7. Utilisation selon l'une quelconque des revendications 1 à 4, dans laquelle la maladie ou l'état pathologique est choisi parmi les anomalies hémostatiques de la fonction glomérulaire, vasculaire et tubulaire ; les troubles de la fonction vésicale et de la relaxation réflexe pour la miction ; et les maladies anales bénignes.
